# EUROPÄISCHE PATENTSCHRIFT

(11) **EP 1 636 594 B1**
(45) Veröffentlichungstag und Bekanntmachung des Hinweises auf die Patenterteilung: **24.08.2011**
(21) Anmeldenummer: 04738662.8
(22) Anmeldetag: 04.06.2004
(51) Int. Cl.: G01N 33/76, C07K 14/59

(54) **VERFAHREN UND MITTEL ZUR BESTIMMUNG VON DEFINIERTEN ZUSTÄNDEN ODER VERÄNDERUNGEN IN DER UTERUSSCHLEIMHAUT ODER IM EPITHEL ANDERER ORGANE**
METHOD AND MEANS FOR THE DETERMINATION OF DEFINED STATES OR MODIFICATIONS IN THE MUCUS OF THE UTERUS OR IN THE EPITHELIUM OF OTHER ORGANS
PROCEDE ET MOYEN PERMETTANT DE DETERMINER DES ETATS OU MODIFICATIONS DEFINIS DANS LES MUQUEUSES DE L'UTERUS OU DANS L'EPITHELIUM D'AUTRES ORGANES

(30) Priorität: 06.06.2003 DE 10325639; 06.06.2003 DE 10325638
(43) Veröffentlichungstag der Anmeldung: 22.03.2006
(73) Patentinhaber: Biotype Diagnostic GmbH, 01109 Dresden (DE)
(72) Erfinder: ALEXANDER, Henry, 04299 Leipzig (DE); ZIMMERMANN, Gerolf, 04207 Leipzig (DE)
(74) Vertreter: Vossius & Partner
(86) Internationale Anmeldenummer: PCT/DE2004/001210
(87) Internationale Veröffentlichungsnummer: WO 2004/109292

(56) Entgegenhaltungen:
- EP-A- 1 321 768
- WO-A-2004/058999
- US-B2- 6 514 708
- BERGER P ET AL: "THE ISOBM TD-7 WORKSHOP ON HCG AND RELATED MOLECULES" TUMOR BIOLOGY, KARGER, BASEL, CH, Bd. 23, Nr. 1, Januar 2002 (2002-01), Seiten 1-38, XP009032819 ISSN: 1010-4283
- LUNDIN M ET AL: "Tissue expression of human chorionic gonadotropin beta predicts outcome in colorectal cancer: A comparison with serum expression" INTERNATIONAL JOURNAL OF CANCER, NEW YORK, NY, US, Bd. 95, Nr. 1, 20. Januar 2001 (2001-01-20), Seiten 18-22, XP002286298 ISSN: 0020-7136
- BELLET D ET AL: "MALIGNANT TRANSFORMATION OF NONTROPHOBLASTIC CELLS IS ASSOCIATED WITH THE EXPRESSION OF CHORIONIC GONADOTROPIN BETA GENES NORMALLY TRANSCRIBED IN TROPHOBLASTIC CELLS" CANCER RESEARCH, AMERICAN ASSOCIATION FOR CANCER RESEARCH, BALTIMORE, MD, US, Bd. 57, 1. Februar 1997 (1997-02-01), Seiten 516-523, XP002040116 ISSN: 0008-5472
- TAKAHASHI K ET AL: "CLINICAL USEFULNESS OF DETERMINATION OF ESTRADIOL LEVEL IN THE MENSTRUAL BLOOD FOR PATIENTS WITH ENDOMETRIOSIS" ACTA OBSTETRICA ET GYNAECOLOGICA JAPONICA - NIHON SANKA FUJINKA GAKKAI ZASSHI, XX, XX, Bd. 41, Nr. 11, November 1989 (1989-11), Seiten 1849-1850, XP001098384 ISSN: 0300-9165

## Beschreibung

Die Erfindung betrifft Verfahren und Mittel zur Bestimmung von definierten Zuständen oder Veränderungen in der Uterasschleimhaut oder im Epithel anderer Organe, insbesondere zur Diagnostik der Schwangerschaft und deren Störungen sowie die Diagnostik des Geburtsbeginnes und zum Festsstellen optimaler Implantationsbedingungen im Uterus, und die Diagnostik von physiologischen und pathologischer Epithelzuständen (Karzinom). Anwendungsgebiet ist die Medizin, insbesondere die Gynäkologie mit den Fachgebieten Reproduktionsmedizin und Geburtsmedizia.

Humanes Choriongonadotropin (Chorionic Gonadotrophin hCG) ist ein Glykoprotein und besteht aus zwei Untereinheiten αhCG und βhCG in nicht konvalenter Bindung (1). Für die Untereinheit αhCG ist ein Gen bekannt (Chromosom 6q21.1-q 23). Für die Untereinheit βCG sind 7 Gene β8, β7, β6, β5, β3, β1 und β2 bekannt (Chromosom 19q13.3).

Während der Schwangerschaft werden durch den Trophoblasten in der Gebärmutter größere Mengen hCG-Dimer und freie αhCG- und βhCG-Moleküle gebildet und in das Blut sezemiert. Embryonales trophoblastäres Gewebe exprimiert fast ausschließlich hCG β5, β8 und β3. Diese βhCG-Untereinheiten werden daher auch trophoblastäres βhCG (tβhCG) oder Typ II-βhCG genannt.

Aber auch in einigen nicht-trophoblastären Geweben wird hCG bzw. seine Untereinheiten in geringen Mengen exprimiert (2-6). Nicht-trophoblastäres Gewebe, wie z. B. Mamma, Lunge, Prostata, Blase, Colon, exprimieren fast ausschlieBlich hCG β7 und β6. Diese βhCG-Untereinheiten werden daher auch als nicht-trophoblastäres βhCG oder Typ I-βhCG bezeichnet (7).

Auch im Blut nichtschwangerer, gesunder Menschen werden daher hCG-Konzentrationen von hCG bis 1000 pg/ml und von βhCG bis 100 pg/ml beobachtet (8, 9). Höhere βhCG-Serumwerte deuten auf einen gonadalen oder nicht gonadalen Tumor hin und kennzeichnen eine ungünstige Prognose, wie bei Lungen-, Blasen-, Prostata-, Colon-, Nierenzell- und Mammakarzinom beschrieben (5, 10-14).

Während die Untereinheiten des Typ II-βhCG (β5, β8 und β3) an Position 117 (Exon 3) der Aminosäuresequenz ein Aspartat (Asp, D) enthalten, enthält Typ I-ßhCG (β7 und β6) an

Position 117 ein Alanin (Ala, A). Das βhCG-Gen β6 ist ein Allel von β7 mit Differenzen in der 5'-nichttranslatierenden Sequenz des Promotor (Exon 1) und in der translatierenden Sequenz (Exon 2) der βhGG-Untereinheit. Nur die Gene β8, β7, β6, β5 und β3 codieren und exprimieren ein βhCG-Proteinmolekül von 145 Aminosäuren (Exon 2 und Exon 3). Die Gene hCG β1 und β2 können zwar auch in einigen Geweben transkribiert werden, codieren aber ein Protein von nur 132 Aminosäuren mit unterschiedlicher Sequenz zum βhCG (15-17).

Das hCG Molekül ist gut charakterisiert durch bekannte Standardpräparationen von bisher 24 monoklonalen hCG-Antikörpern der "International Society of Oncodevelopmantal Biology and Medicine" (ISOBM), die verschiedene definierte Bpitope auf der αhCG-Untereinheit (α1-α7, n=7), auf der βhCG-Untereinheit (β1-β9, n=9), auf dem βhCG-core fragment (cfβ10-cfβ13, n=4) und auchkanformationsabhängige Epitopen des intakten αß-Heteodimers (αß1 - αβ4, n=4) spezifisch und in hoher Epitopaffinität erkennen (18-20).

Die Antikörper erkennen bevorzugt Epitop in der räumlichen Aminosämeanordnung des hCG-Moleküls, d. h. seiner Tertiär- und Quartälstruktur (17, Fig. 2 und 3 in18) Deswegen werden für die Präparation der Hybridome zu Bildung obengenannter monoklonale ISOBM-Antikörper weitgehend WHO-Referenzhonnon-Präparationen des hCG respektive βhCG der Inter-national Federation of Clinical Chemistry (IFCC) als adäquate Antigendeterminanten verwendet Aufnahmen bilden die βhCG-Epitope β8 (AS 137 bis 144) und β9 (AS 109 bis 116) des C-terminalen Endes (CTP) sowie ein Anteil des βhCG-Epitopes β1 (AS 1 bis 10) am N-terminalen Ende von βhCG, die randständig an der Oberfläche des hCG-Dimers in Cystinknoten-Struktur und weitgehend unbeeinflusst von der Tertiärstruktur ein immunologisches Antigenpotential darstellen (21, 22). Diese linearen βhCG-Epitop-Abschnitte um etwa AS 1-16, AS 108-123 und AS 137-144 werden von monoklonalen βhCG-Antikörpern erkannt, die mit der Methode der carriergebundenen synthetischen Peptide als Antigendeterminante der ISOBM-Antikörper produziertwerden (18,19,21,22).

Für die Antigenregionen β1, β8, β9 des βhCG Moleküls (Fig. 3 in 18) sind bereits ISOBM-βhCG-Antikörper dargestellt worden, die auf der bekannten Aminosäuresequenz der trophoblastären (oder plazentaren) βhCG-Untereinheit beruhen. Besonders die Aminosäuresequenz um 108-123 des C-terminalen Endes von βhCG (βhCG-CTP), charakterisiert als Epitop β9, zeigt eine hohe Antigenizität (21). In etwas geringerem Maß trifft es auch für die Aminasäuresequenz um 1-16 als Anteil des Epitopes β1 zu (23). Antikörper, mit synthetischen Peptiden gegen diese Peptidsequenzen erzeugt, erkennen die native trophoblastäre βhCG-Untereinheit spezifisch (18, 19, 21, 23, 24).

In der Vergangenheit sind verschiedene Studien mit dem Ziel durchgeführt worden, die βhCG-Transkripte in verschiedenen normalen und neoplastischen Geweben nichttrophoblastärer Herkunft mit semiquantitativer Methode nachzuweisen (5, 12, 13, 25). Diese Methoden zeigen, daß βhCG in normaler Plazenta (26), gesunden Testes (6), aber auch neoplastischen Testes (27) und neoplastischern Blasengewebe (28) transkribiert wird In diesen Studien wird jedoch nicht zwischen Typ I-βhCG und Typ II-βhCG unterschieden.

Dafür sind unterschiedliche Testkits vorgeschlagen worden, die mit unterschiedlicher Epitop-Spexifität arbeiten (29) und die Bestimmung des trophoblastären Gesamtmoleküls αßhCG (Gesarnt-hCG) oder der einzelnen Untereinheiten des Moleküls βhCG und αhCG ermöglichen (18). Speziell für die Schwangerschaftstests und Diagnostik von Blasenmole und Chonon-karzinorn werden Verfahren angewendet, in denen das heterodimäre trophoblastäre Gesamtmolekül hCG allein (Cesarnt-thCG) oder in der Summe mit der freien Untereinheit des trophoblastären βhCG (Gesamt-thCG plus tβhCG ) oder als freie tβhCG-Untereinheit allein bestimmt werden. Die Heterogenität des trophoblastären hCG im biologischen Material (intaktes αβ-Heterodimer, freies αhCG, freies tβhCG, tβhCG core-Fragment, nicked thCG, nicked tβhCG), bezogen auf die Bindung an den jeweilig verwendeten Antikörper, erschweren die genaue Bestimmung und die Standardisierung eines möglichen Nachweisverfahrens für das eptithal hCG. Eine zusätzliche Heterogenität der tβhCG-Bestimmung in der sekretorischen Zyklusphase und frühen Schwangerschaft kann im gelingen Grad auch zwischen den vier nativen, hyperglykosylierten oder desialylierten Kohlenhydratseitenketten des C-terminalen Endes (CTP) von tβhCG (Aminosäure 120 bis 145) auftreten, wie sie different in der frühen bis mittleren Schwangerschaft und bei Chorion-Karzinom im trophoblastären βhCG beobachtet worden sind (31, 33-37).

Die bisher bekannten Schwangerschaftstests haben den Nachteil, dass sie häufig falsch positive Ergebnisse liefern.

Die bisher bekannten Schwangerschafstests können verminderte hCG-Konzentrationsmessungen während der Extrauteringravidität (Eileiterschwangerschaft) oder hCG-Titer bei Patientinnen nach IUD-Einlage nur unzureichend unter dem Aspekt veränderten uterinen Sekretionsvehaltens bewertet werden (38, 39).

Als "Phantom hCG" bezeichnet man ein Phänomen, bei dem ein Schwangerschaftstest im Blut positiv für hCG ist, obwohl keine Schwangerschaft oder ein Tumor im Genitaltrakt vorliegt. Bei der heutigen Diskussion der Phantom hCG Werte geht man davon aus, dass dieses Phänomen auf eine abnormale Interaktion zwischen dem Test und in der Blutprobe der Patientin enthaltenen üregulären Antikörper zurückzuführen ist (54).

Aufgabe der Erfindung ist es, Verfahren und Mittel anzugeben, welche es ermöglichen, definierte Zustände bzw. Veränderungen in der Uterusschleimhaut (Endometrium, Dezidua), aber auch in den Epithelien anderer Organe zu ermitteln. Das Verfahren und die Mittet sollen insbesondere das Feststellen optimaler Implantationsbedingungen im Uterus und eine zuverlässige Diagnostik der Schwangerschaft und deren Störung sowie des Geburtsbeginnes ermöglichen.

Der Erfindung liegt die wissenschaftliche Erkenutnis zngrunde, dass im endometrialen und dem dezidualen Epithel βhCG-Untereinheiten exprimiert werden, welche sich in mehreren Aminosäure-Positionen von den bekannten trophoblastären βhCG-Untereinheiten unterscheiden.

Die Nukleotidsequenz und Proteinsequenz fur die endometriale βhCG-Untereinheit wird hier erstmals in SBQ ID No 7 und SBQ ID No 10 dargestellt (eßhCG oder "Endo").

Die im endometrialen und dem dezidualen Epithel exprimierten βhCG-Untereinheiten werden nachfolgend als endometriales βhCG (eßhCG) bezeichnet. Unsere Ergebnisse zeigen, dass das eßhCG eine endometrialen Variante der Untereinheiten β7 und β6 darstellt, während trophoblastäres βhCG ausschließlich aus den Untereinheiten β5, β8 und β3 gebildet wird.

Gefunden wurden folgende Unterschiede des eßhCG zum bekannten trophoblastären βhCG (tβhCG):

Eine Variation von Aspartat (tβhCG) in der Aminosäureposition 117 des C-terminalen Endes von βhCG (βhCG-CTP) zu Alanin (eßhCG).

Weitere Variationen wurden in Position 2 und Position 4 des Exon 2 gefunden. Das trophoblastäre βhCG (tβhCG) hat an Position 2 ein Lysin und an Position 4 ein Prolin. Das endometriale βhCG (eßhCG) hat an Position 2 ein Arginin und an Position 4 ein Methionin.

Unter endometrialern βhCG (ehCG) wird nachfolgend ein βhCG verstanden, welches zumindest eine der zuvor genannten Variationen aufweist

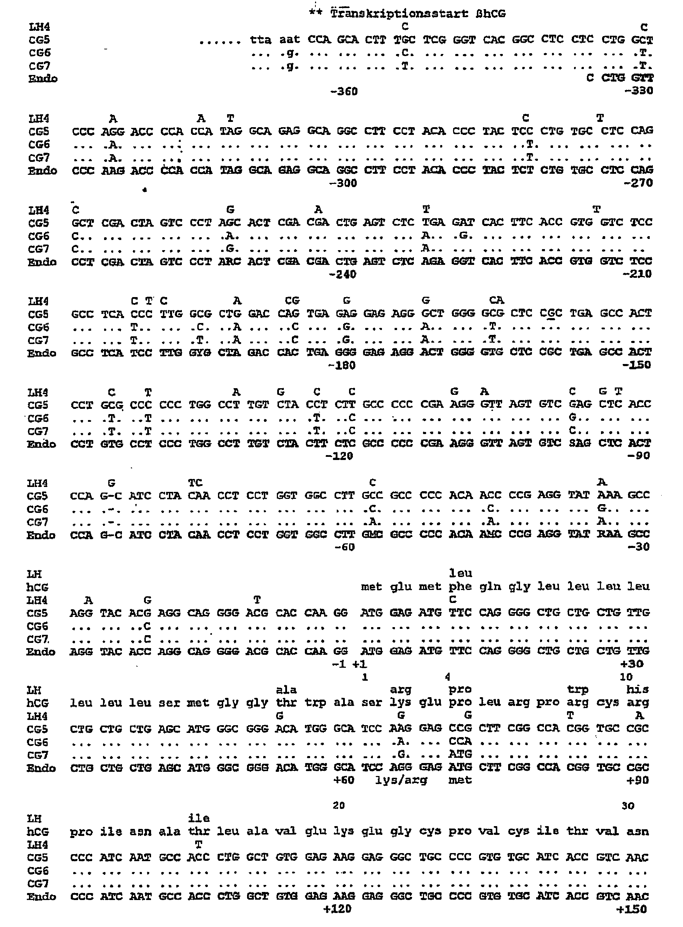

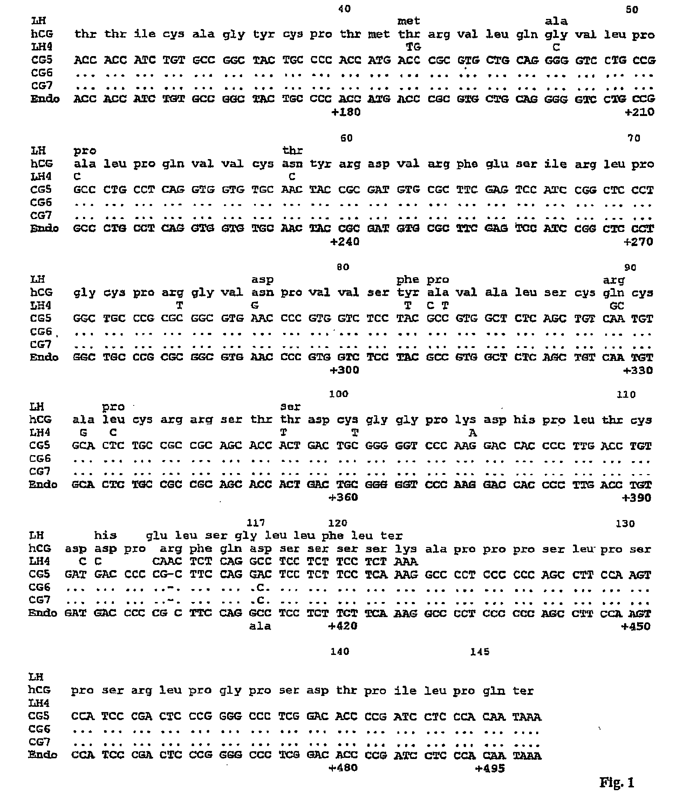

In Fig. 1 wird ein Alignment der Sequ des eßhCG ("Endo" mit den Sequenz des trophoblastären βhCG-Untereinheit tβhCG β5 und den bekannten nicht-trophoblastaren βhCG-Untereinheiten β6 und β7, sowie des hypophysären ßLH ß4 (ß-Untereinheit des luteuisierenden Hormons) gezeigt.

Die Nukleotidsequenz des endormetrialen eßhCG (SEQ ID No 7) unterscheiden sich auch von den bekannten nicht-trophooblastären βhCG-Untereinheiten β7 (SEQ ID No 5) und β6 (SEQ ID No 6), insbesondere im Promotorgen des Exon 1, aber auch in Exon 2 am Expressionsort der Aminosäureposition 2 und 4 (Fig. 1). In der nach Genexpression resultierenden Aminosäuresequenz (SEQ ID No 10) repräsentiert sich das endometriale eßhCG als Variante des epithelialen Typ 1- βhCG des βhCG β7-Proteins (SEQ ID No 9) mit drei differierenden Aminosäuren an Position 2, 4 und 117 zwischen dem endometrialen respektive dezidualem eßhCG und dem herkömmlichen trophoblastären tβhCG (SEQ ID No β). Die Aminosäuresequenz des endometrialen eßhCG (SBQ 1D No 10) differiert zur Sequenz des hypophysären βLH β4 (SEQ ID No 11) beträchtlich (Fig. 1).

Weiterhin beruht die Erfindung auf der wissenschaftlichen Erkenntnis, dass die Ursache für die falsch positiven Ergebnisse, der aus dem Stand der Technik bekannten Tests ist, dass sie nicht zwischen den trophoblastären Sekretionsleistungen des Embryos in Form von tβhCG und den Sekretionsleistungen die mit der sekretorischen Transformation und uterinen Dezidualisierung des Endometriums sowie bei der Epitheldifferenzierung entstehen (eßhCG) unterscheiden.

### Verfahren:

Basierend auf dieser Erkenntnis wird erfindungsgermäß die Aufgabe gelöst durch ein Verfahren zur Bestimmung von definierten Zuständen oder Veränderungen in der Uterusschleimhaut oder im Epithel anderer Organe, bei dem in einer Korperflüssigkeitsprobe und/oder Gewebeprobe die Konzentration von endometrialem βhCG bzw. nicht-trophoblastärem βhCG spezifisch bestimmt wird Unter spezifischer eßhCG-Bestimmung wird dabei verstanden, dass zwischen endometrialem βhCG und trophoblastärem ehCG unterschieden wird.

Die Bestimmung der Konzentration erfolgt bevorzugt in einer Probe (in Form von Sekreten, Perfusionsflüssigkeit, Zellen oder Gewebe) aus peripheren Blut, Serum, Menstrualblut, Lochia, Fruchtwasser, Urin, Speichel, Augenkammerwasser sowie Sekreten des Urogenital-(incl. Uterus, Zervix, Vaginalproben), des Gastrointestinal- (incl. Mundschleimhaut) und des Respirationstraktes sowie des zentralen Nervensystems (incl. Liquor).

Bevorzugt wird im erfindungsgemäßen Verfahren, neben der Konzentration von endometrialem βhCG bzw. nicht-trophoblastärem ßhCG auch, die Konzentration von trophoblastärem βhCG (tßhCG), Gesamt-hCG oder Gesamt-hCG bestimmt.

Die Bestimmung von trophoblastäreim βhCG (tßhCG), Gesamt-ßhCG, oder GesamthCG erfolgt bevorzugt nach bekannten Methoden (18,19,21,22,23" 24,29,30,31,54).

Da diese bisher bekannten Methoden nicht zwischen tβhCG und ehCG unterscheiden, ermöglicht die erfindungsgemäße spezifische Bestimmung des eßhCG erstmals auch eine Aussage darüber, ob das mit diesen Tests bestimmte tβhCG tatsächlich tβhCG ist, und wie hoch der Anteil an tßhCG und eßhCG ist.

Der Anteil des tßhCG ergibt sich aus der Konzentration des Gesamt-hCG / ßhCG oder GesamthCG abzüglich des gemessenen eßhCG bzw. nicht-trophoblastären ßhCGs. Vorteilhaft eignet sich das erfindungsgemäße Verfahren insbesondere zur Diagnose der Aumahmcbereitschaft (Rezeptivität) der Uternsschleimhaut für eine befruchtete Eizelle und ermöglicht damit das Feststellen optimaler Implantaiionsbedingungen im Uterus.

Es wurde nun festgestellt, dass die Expression von eßhCG in der Uterusschleimhaut (Endometrium) nötig ist, um das erfolgreiche Einnisten einer befruchteten Bizelle zu ermöglichen. Eine beginnende eßhCG-Expression oder eine ehCG ist ein Zeichen für die Aufnahmebereitschaft der Uterusschlennhaut für eine befruchtete Eizelle.

Die Diagnose der Aufnahmebereitschaft der Uterasschleimhaut (Implantationsbedingung) für eine befruchtete Eizalle erfolgt bevorzugt prospektiv, in dem einer Patientin in der frühen Lutealphase Gewebe vom Endometrium oder von der Zervixschleimhaut (Mundschleimhaut), Vaginal-, Zervix- oder Uterussekret oder Serum, Plasma und Peripherblut entnommen wird und in dieser Probe die nicht-trophoblastäre oder endometriale βhCG-Konzentration bestimmt wird Aus der Höhe der ermittelten Expression können dann Rückschlüsse auf die Aufnahmebereitschaft der Gebärmutter für einen Embryo im aktuellen oder prognostische Aussagen für den Folgezykhis getroffen werden.

Dazu werden bevorzugt einige Tage nach der Ovulation Zellen mit einem Minikatheter aus der Gebärmutterhöhle, mit einem Wattebausch aus dem Zervikalkanal oder mit einem Holzspatel von der Mundschleimhaut gewonnen bzw. peripheres EDTA- bzw. Heparinblut entnommen. In den aufgenommenen Zellen wird die nicht-trophoblastäre oder endometriale βhCG-Konzentration bestimmt,

Für die prospektive Diagnostik der Embryorezeptivität in der frühen Sekretionsphase des aktuellen Zyklus werden bevorzugt Gewebeproben des Endometrims, aus der Endocervix, der Mundschleimhaut oder anderer ausgewählter Epithelien wie auch Cervix-/ Vaginalsekret oder endometriales Sekret nach Abstrich oder als Perfusat untersucht, um über die Qualität der zu erwartenden sekretorischen Transformation und Rezeptivität des Endometriums (z. B. für die Entscheidung eines Embryotransfers nach In-vitro-Fertilisation im hormonell stimulierten Zyklus) zu befinden.

Über die Aussage der Implantationsbedingungen im vorausgegangenen Zyklus lassen sich Prognosen über die Implantationsbedingungen, d. h. die Aufnahmebereitschaft der Gebanoutter für eine befruchtete Eizelle oder einen Embryos, im Folgezyklus machen.

Eine weitere bevorzugte Verwendung des Verfahrens ist daher die Anwendung zur retrospektiven Diagnostik der Rezeptivität der Uterusschleimhaut. Unter retrospektiven Implantationsdisgnostik wird im Sinne der vorliegenden Erfindung verstanden, die sekretorische Transformation des Endometriums des vorangegangenen Zyklus zu detektieren um Voraussagen für die Rezeprivität eines folgenden Zyklus zu treffen. Diese lassen eine ungestörte zeit- und funktionsgerechte Bierstocks-Gebärmutter-Beziehung erkennen.

Das erfindungsgemäße Verfahren kann damit die invasive Methode der Strichsbrasio in ihrer Aussage ergänzen oder ersetzen.

Mit der Bewertung und Quantifizierung der spezifischen epithelialen endometrialen hCG-Sekretion (ehCG) in Körperflüssigkeiten und Zell-(Gewebe-)homogenaten der frühen, mittleren und späten Sekretionsphase des menstruellen Zyklus können optimale Implantationsbedingungen wie auch mögliche Fertilisationsstörungen sowohl prospektiv als auch retrospektiv unter dem Aspekt der endometrialen Diagnostik und Therapiekontrolle erfasst werden.

Bei der retrosgektiven Diagnostik der Rezeptivität der Uterusschleimhaut kann prinzipiell, wie zur prospektiven (vorbereitenden) Implantationsdiagnostik verfahren werden. Bevorzugt erfolgt die Analyse der βhCG-Konzentration jedoch in einer Probe von Blut oder einer Probe von im Menstrualblut enthaltenen Zellen.

Im Menstrualblut sind ausreichend Zellen des Endometrium vorhanden, die eine Bestimmung der βhCG-Konzentration ermöglichen.

Der Vorteil der retrospektiven Diagnostik im Menstualblut gegenüber der zuvor beschriebenen prospektiven Methode, liegt darin, dass sie nicht invasiv ist, Es muss weder Peripherblut noch eine Gewebeprobe aus der Gebärmuttter entnommen werden. Trotzdem. kann mit dieser Methode eine zeit- und funkdonsgerechte Umwandlung des Endometriums in der Sekretionsphase des Zyklus erkennt werden, was gleichzeitig Ausdruck einer ungestörten Regulationsfunktion auf den Bbenen Hypothalamus/Hypophyse, Eierstock und Uterus ist Menstrualblut kann wie Peripherblut nach Zentrifugation zur Abtrennung von Zellen und Stroma für die direkte Messung der endometrialen βhCG-Sekretion mit spezifischen Antikörpern im ELISA-Test eingesetzt werden. Das Menstrualblut wird nach spontanem Zyklus, nach Hormontherapie, nach In vitro-Fertilisation (IVF) und Embryotransfer (BT) ohne erfolgreiche Implantation sowie bei vorgesehener Diagnostik des Zyklus bei Kinderwunschpatientinnen und bei Patientinnen mit gynäkologischen Erkrankungen wie Myom, Endometriose, Endometrium- und Cervixkarzinom gewonnen. Dabei ist die parallele Gewinnung von Peripherblut zum selben Zeitpunkt als Heparinblut oder als Serum zum Ausschluss eines erhöhten unspezifischen Serum-hCG-Wertes unbedingt erforderlich.

Ist die Konzentration des eßhCG bzw. nich-trophoblastären ßhCG im Menstrualblut gegenüber der Konzentration im Peripherblut erhöht, kann von einer lokalen Bildung im Bndametrium ausgegangen werden. Eine hohe ehCG Konzentration ist dabei Ausdruck einer physiologischen und eine fehlende oder niedrige ehCG Konzentration Ausdruck einer pathologischen Funktion des Endometriums. Da im Peripherblut kein tßhCG enthalten ist kann für die Bestimmung im Peripherblut allein ein üblicher ß-hCG-Nachweis (ELISA, MBIA u. a. - Literafurstellen 18, 19, 21, 22, 23, 24, 28, 29, 30, 54), der nicht zwischen tßhCG und eßhCC unterscheidet, verwendet werden.

Bei der prospektiven oder der retrospektiven Diagnose der Aufnahmebereitschaft der Uterusschleimhaut ist eine Unterscheidung zwischen endometrialen und trophoblastären ßhCG nicht zwingend notwendig, da noch keine Schwangerschaft vorliegt und damit eine Expression von tßhCG durch einen Trophoblasten ausgeschlossen werden kann. Die Erfindung umfasst daher auch die Verwendung eines Verfahrens bei dem die Konzentration an Gesamt-hCG / ßhCG oder Gesamt-ßhCG zur Diagnose der Aufnahmebereitschaft der Uterusschleimhaut für eine befruchtete Eizelle bestimmt wird. Bei diesem Verfahren erfolgt die Bestimmung von hCG bevorzugt mit Antikörpern, die αβ hCG die bekannten ß7, ß6 oder ß6e hCG-Untereinheiten erkennen. Diese Antikörper müssen nicht spezifisch für eßhCG sein, dh. sie können auch tßhCG erkennen.

Als derartiger Antikörper wird bevorzugt ein bekannter polyklonaler oder monoklonaler anti-αßhCG oder ein auti-ßhCG-Antikörper verwendet. Dieser Antikörper erkennt bevorzugt ein

Epitop ausgewählt aus der Gruppe der Epitope ß1 bis ß9 (besonders bevorzugt ß2 bis ß8) der ßhCG-Unterreinheit, der Epitope cfß1 bis cfß13 auf dem ßhCG-core Fragment und der konformationsabhängigen Epitope αß1 bis αß4 des intakten αß-Heterodimers nach der Klassifizierung der "International Society of Oncodevelopmental Biology and Mediane" (ISOBM) (18-20). Bezogen werden können derartige Antikörper beispielsweise von der Firma BIOTREND Chemikalien GmbH, Köln, Deutschland oder Serotec Düsseldorf, Deutschland

Die Diagnose der Aufnahmebereitschaft der Uterusschleimhaut für eine befruchtete Eizelle wird bevorzugt retrospektiv mit einer Probe von Menstrualblut oder einer Probe von im Menstrualblut enthaltenen Zellen durchgeführt. In der parallelen Abnahme des Peripherblutes ist der gemessene hCG Wert vernachlässigbar klein zum hCG-Wert im Menstrualblut

Während der Menstruation kann eine Schwangerschaft und damit eine Expression von tßhCG durch einen Trophoblasten ausgeschlossen werden. Eine Unterscheidung zwischen eß-hCG und tßhCG kann daher im Menstrualblut auch ohne spezifischen eßhCG-Nachweis erfolgen.

Die Anzneldung beschreibt daher auch ein Verfahren zur Bestimmung von definierten Zuständen oder Veränderungen in der Uterusschleimhaut oder im Epithel anderer Organe, bei dem die Bestimmung der Gesamt-hCG-, ßhCG- oder GesamthCG / -ßhCG-Konzentration wie oben beschrieben in einer Probe aus Menstmalblut erfolgt.

Entgegen der allgemeinen Auffassung ist nicht tßhCG das erste in der Frühschwangerschaft nachgewiesene ßhCG, sondern endometriales oder deziduales eßhCG.

Vorteilhaft eignet sich damit das erfindungsgemäße Verfahren auch dazu, die Aussagekraft bestehender Schwangerschaftstests zu verbessern. In den bekannten Schwangerschaftstests wird nur das ßhCG bestimmt, welches vom Trophoblasten gebildet wird, bzw. unspezifisch Gesamt-ßhCG bestimmt. Da ehCG bereits in der gut ausgebildeten Sekretionsphase des Endometriums gebildet wird und mit der Embryoimplantation das deziduale hCG verstärkt freisetzt wird, ist es mit dem erfindungsgemäßen Verfahren möglich, zu einem früheren Zeitpunkt eine Schwangerschaftsdiagnose zu stellen, da hierbei durch die frühen Schwangerschaftsabläufe das ehCG nicht wie bei einer Menstruation nach außen sondern durch einen Flow in den Blutkreislauf abgegeben wird.

Da die bekannten Tests auch keine Aussage darüber machen, ob der Trophoblast sich erfolgreich in die Gebärmutter eingenistet hat, führen sie häufig zu falsch positiven Ergebnissen

Im Unterschied zu den bekannten Schwangerschaftstests, in denen die Heterogenität des ßhCG nicht berücksichtigt wird, wird erfingdungsgemäß die Konzentration von endometrialem ßhCG bzw. nicht-trophoblastärem ßhCG spezifisch bestimmt Dies ermöglicht eine Aussage über die Sekretionsleistung und Rezeptivität der Uterusschleimhaut, welche Vorraussetzung für eine erfolgreiche Schwangerschaft ist. Das erfindungsgemäße Verfahren führt daher zu einer zuverlässigeren Schwangerschaftsdiagnose gegenüber den bekannten Verfahren. Das erfindungsgemäße Verfahren ermöglicht auch die Unterscheidung zwischen einer Extrauteringravidität oder eines frühen Schwangerschaftsverlusts und einer intrauterinen Schwangerschaft. Bei einer intrauterinen Implantation des Embryos ist die Expression von eßhCG höher, als bei einer extrauterinen Implantation. Die Diagnose einer Extrauteringravidät erfolgt bevorzugt durch Analyse einer Serumprobe. Eine niedrige Konzentration von eßhCG im Serum bei normalem tßhCG ist ein Zeichen für eine Extrauteringravidät.Bei einem frühen Schwangerschaftsverlust ist eßhCG vorhanden, es fehlt jedoch eine tßhCG- Expression.

Bevorzugt wird zur Schwangerschaftdiagnose, neben der Konzentration von endometrialem ßhCG bzw. nicht-trophoblastärem ßhCG auch die Konzentration von trophoblastärem ßhCG (tßhCG), Gesamt-ßhCG oder GesamthCG nach den oben genannten bekannten Methoden bestimmt.

Die erfindungsgemäße spezifische Bestimmung des eßhCG ermöglicht erstmals auch eine Aussage darüber, ob das mit den bekannte Methoden bestimmte ßhCG tatsächlich tßhCG ist, und wie hoch der Anteil an tßhCG und eßhCG ist Damit kann erstmals sicher diagnostiziert werden, ob eine Schwangerschaft tatsächlich vorliegt oder nicht. Eine Schwangerschaft ist dann vorhanden, wenn sicher thCG nachgewiesen werden kann. Da ehCG epithelialen Ursprunges ist und von der Frau stammt, ist ein kurzfristiger mit nach dem Stand der Technik durchgeführter hCG Nachweis bei ausgebliebener Regelblutung nicht gleichbedeutend mit einer Frühschwangerschaft. Nach dem Stand der Technik werden häufig frühe Schwangerschaftsverluste als Schwangerschaft fehlinterpretiert. Gleiches gilt für den nach dem Stand der Technik durchgeführter hCG Nachweis in der zweiten Zyklushälfte bei Trägerinnen einer Kupferspirale (Cu-IUD). Auch hier besteht keine Schwangerschaft, sondern das alterierte Endometrium reagiert mit eine ehCG Freisetzung.

Mit der differenzierten Bestimmung der Konzentration des ehCG und deren Relation zu thCG kann erstmals auch diagnostisch unterschieden werden, ob eine Schwangerschaftsstörung bedingt ist durch eine Veränderung der Dezidua oder der trophoblastären-embryonale/fetalen Binheit.

Das erfindungsgemäße Verfahren eröffnet auch die Möglichkeiten, die Schwangerschaft während ihres Verlaufs zu überwachen und eine Aussage über mögliche Schwangemchaftsstörungen bzw. den Erfolg einer Schwangerschaft zu machen.

Eine ungestörte Schwangerschaft ist gekennzeichnet durch hohe ehCG Werte im Peripherblut und in Proben des Genitaltraktes (Abstrich, Sekret, Gewebe). Bei erniedrigten ehCG Werten besteht eine Abortneigung. Durch das Verfahren kann eine Abortneigung frühzeitig diagnostiziert werden und umgehend eine Therapie eingeleitet werden. Danach kann das Verfahren zur Therapiekontrolle eingesetzt werden. Dabei ermöglicht das erfindungsgemäße Verfahren vorteilhaft die Differenzierung zwischen einer Störung der Dezidua von einer trophoblastären/embryonalen Störung bei beginnendem Abort. Bei einer trophoblastären/embryonalen Störung ist das tßhCG erniedrigt.

Auch andere Schwangerschaftsstörungen wie intrauterine Wachstumsretardierung und Präeklampsie zeigen unphysiologische, d. h. erniedrigte, ehCG Werte, welche mit dem erfindungsgemäßen Verfahren diagnostiziert werden können.

Die Bestimmung des ehCG im Serum und aus Proben des Genitaltraktes (Abstrich, Sekret, Gewebe) kann vorteilhaft für das Frühgeburtenscreening und die Bestimmung des Geburtsbeginns eingesetzt werden. Erhöhte eßhCG-Werte in Sekreten des Urogenitaltrakts, insbesondere Vaginal- und Zervikalsekreten, und erniedrigte Werte im Serum zeigen eine Frühgeburt bzw. am Ende der Schwangerschaft den Geburtsbeginn an.

Die spezifische Bestimmung der ehCG Konzentration im Fruchtwasser ermöglicht vorteilhaft die Diagnostik der Deziduafunktion während der Schwangerschaft. Nach der Geburt kann die

Dezidua-Funktion vorteilhaft auch durch spezifische Bestimmung der ehCG Konzentration in der Lochia nachträglich bestimmt werden. Eine hohe eßhCG-Konzentration ist in beiden Fällen ein Zeichen für eine gesunde Funktion der Dezidua. Eine verminderte tßhCG-Konzentration ist jedoch ein Hinweis auf eine Schwangerschaftspathologie.

Das erfindungsgemäße Verfahren der spezifischen Bestimmung von eßhCG bzw. nicht throphoblastären ßhCG, eignet sich auch zur Beurteilung der Effektivität einer kontrazeptiven Methode. Ein Ausbleiben, Absinken oder die zeitliche Verschiebung der ehCG Sekretion ist dabei ein Zeichen für die Qualität der kontrazeptiven Potenz und ermöglicht die Klassifizierung einer Methode.

Dazu wird die eßhCG-Konzentration in Proben (Sekret, Spülflüssigkeit, Zellen, Gewebe) des Endometriums bevorzugt in einem ELISA mit eßhCG-spezifischen Antikörpern bestimmt

Wird kein eßhCG durch das Endometrium gebildet, ist dies ein Zeichen dafür, dass das Endometrium nicht aufnahmebereit für eine befruchtete Eizelle ist. Es besteht somit Schutz vor einer Schwangerschaft.

Das erfindungsgemäße Verfahren kann vorteilhaft auch zur Differenzierung zwischen physiologischen und pathologischen Epithelzuständen eingesetzt werden. Dabei signalisiert eine apitheliale ehCG-Expression oder eine Expression von nicht-trophoblastärem ßhCG (ß7, ß6) physiologische Verhältnisse, während der Nachweis von tßhCG (ß5, ß8, ß3) in einer Zell- oder Gewebeprobe einen Hinweis auf einen pathologischen Epithelprozess, z. B. einen Tumor, eine beginnende Dedifferenzierung oder eine beginnende karzinomatöse Entartung oder ein Karzinom gibt.

Pathologische Epühelprozesse, welche auf diese Weise mit dem erfindungsgemäßen Verfahren insbesondere diagnostiziert werden können, sind Endometriosen, Myome Schilddrüsenerkrankungen sowie Karzinome des Endametriums, des Eierstocks und des Peritoneums.

Die Bestimmung der eßhCG-Expression und die parallele Bestimmung des Gesamt- hCG /ßhCG oder GesamthCG erfolgt dabei bevorzugt im biologischen Material der Desquamation des endometrialen Gewebes nach Separation der epithelialen Zellen von den Strömazellen, den peripheren mononukleären Blutzellen und den mononukleären Immunzellen des endometrialen Epithels.

Durch das erfindungsgemäße Verfahren ist es damit möglich, eine regelrechte Differenzierung epithelialer Organe zu unterscheiden von einer Fehldifferenzierung and beginnenden karzinomatösen Entartung. Dies wird möglich, da auf der Ebene der Transkription und Translation zwischen dem ß7 hCG und dem tumorspezifischen ß5 hCG differenziert werden kann. Die Konzentrationen und ihre Relationen beider hCG Typen geben eine Aussage über die Epithelgesundheit bzw. deren Störung im Sinne einer Entdifferenzierung. Auch die bösartige Potenz und die Prognose einer Tumorerkrankung könnte davon abgeleitet werden.

Durch die Erkenntnis, dass hCG auch ein epitheliales Hormon ist, das vom Epithel der inneren Oberfläche sezerniert wird, ist der Nachweis eines hCG im Serum von gesunden, nicht-schwangeren Patientinnen und Patienten ohne Nachweis eines Tumors nicht überraschend. Gegenwärtig wird dieser nicht erklärbare hCG Nachweis bei Patientinnen/Patienten ohne Schwangerschaft und Tumor als das sogenannte "Phantom hCG" noch mit dem Vorhandensein von irregulärem Antikörpern in Verbindung gebracht. Durch das erfindungsgemäße Verfahren der spezifischen Bestimmung von eßhCG bzw. nichtthrophoblastären ßhCG können diese Fälle von "Phantom hCG" als eine physiologische Spielvariante einer epithelialen Funktionsleistung erklärt werden. Durch das erfindungsgemäße Verfahren kann eine physiologisch erhöhte Expression eßhCG bzw, nichtthrophoblastären ßhCG (ß7, ß6) von einer·erhöhten tßhCG (ß5, ß8, ß3)-Expression in einem Karzinom unterscheiden werden. Unnötige Chemotherapien und langwierige kostenaufwendige Überwachungen dieser Patienten erübrigen sich somit.

Die Bestimmung der Konzentration von endometrialem ßhCG (eßhCG) erfolgt semiquantitativ oder quantitativ. In einer bevorzugten Ausführungsform erfolgt die Konzentrationsbestimmung mit mindestens einem Antikörper, der spezifisch eßhCG bzw. nicht trophoblastäres ßhCG (tßhCG) erkennt, in einem ELISA, Dotblot-, oder Westemblotassay, in einer immunohistochemischen Methode, Flow-Cytometry, oder in einer anderen benannten Antikorper-basierten Methode. In einer alternativen Ausführungsform erfolgt die Bestimmung der Konzentration von endometrialem ßhCG (eßhCG) und die Unterscheidung zu trophoblastärem ßhCG auf der Ebene der RNA-Expression, z. B. durch die bekannten Methoden der RT-PCR, oder z. B. durch die Hybridisierung mit einer Oligonucleotid-Sonde.

Die Erfindung umfasst auch die Antikörper, welche spezifisch eßhCG und nicht tßhCG erkennen. Der Begriff Antikörper im Sinne der vorliegenden Erfindung umfasst dabei neben monoklonalen und polyklonalen Antikörpern auch rekombinante Antikörper und Fragmente, wie z. B. scFv (single-chain-Fragmente) und Fab-Fragmente mit ein. Bevorzugt trägt der Antikörper ein Markermolekül, wie z. B. Biotin, Dioxygenin oder einen Fluoreszenfarbstoff

Die erfindungsgemäßen eßhCG-spezifischen Antikörper erkennen ein Hexa- bis Decapeptide im Bereich der Aminosäurepositionen 2 und 4 (SEQ ID No 3) der Sequenz des eßhCG (SEQ ID No 10). EßhcG-spezifischen Epitopbereiche werden eß9 (SEQ ID No 1) und eß1 (SEQ ID No 3) genannt. Aus diesen Epitop-Bereichen erkenennen die erfindungsgemäßen eßhCG-spezifischen Antikörper ein Biptop, welches die Aminosäurepositionien 2 und 4 umfasst, wie z. B.:

Die erfingsgemäßen eßhCG-spezifischen Antikörper reagieren jedoch nicht mit den entsprechenden tßhCG-Epitopen ß9 und ß1 aus den entsprechenden Bereichen (SEQ ID No.2 und 4) der Sequenz für tßhCG (SEQ ID No. 8), wie z. B.:

Die erfindungsgemäßen Antikörper sind bevorzugt mit einem Peptid ausgewählt aus den Peptidsequenzen gemäß SBQ ID No. 3 und 14 bzw. deren Teilsequenzen generiert und reagieren nicht mit den Kontrollpeptiden für tßhCG gemäß SBQ ID No. 2, 13, sowie 4 und 15.

Bin weiterer Gegenstand der Erfindung ist ein Testkit zur Bestimmung von definierten Zuständen oder Veränderungen in der Uterusschleimhaut oder im Epithel anderer Organe.

Dieses Testkit enthält mindestens einen Antikörper, welcher spezifisch eßhCG erkennt, sowie gegebenenfalls Stabilisatoren, weitere Antikörper, wie z. B. weitere anti-hCG-Antikörper, sekundäre Antikörper, Standards, Puffer, Reagenzien zum Blockieren freier Bindungsstellen (z. B. Magermilchpulver oder bovines Serumalbumin (BSA).

Bevorzugt ist in dem diagnostischen Kit der Antikörper, welcher spezifisch eßhCG erkennt, an einen festen Träger gebunden. Ein solcher fester Träger ist z. B. eine ELISA-Platte, bevorzugt aus Polycarbonat, oder im Falle des Dotblot- oder Westernblot-Assays eine Folie, bevorzugt aus Nitrozellulose.

Die Bindung des Antikörpers an eine ELISA-Platte ermöglicht vorteilhaft die Durchführung eines Sandwich-ELISAs, in dem das Binden des eßhCG an den eßhCG-spezifischen Antikörper durch einen zweiten antihCG-Antikörper detektiert wird Die Bindung des Antikörper an eine ELISA-Platte wird z. B. durch inkubieren der Platte mit einer Antikörperlösung in 50 mmol/Liter Carbonat bei einem pH-Wert von pH 8 bis pH 9 für mindestens eine Stunde und anschließendes Trocknen erreicht

Als zweiter anti hCG-Antikörper wird bevorzugt ein polyklonaler oder monoklonaler antießhCG oder ßhCG Antikörper verwendet, der im Unterschied zu den erfindungsgemäßen eßhCG-spezifischen Antikörpern sowohl das endometriale als auch trophoblastäre hCG erkennt. Dieser Antikörper erkennt bevorzugt ein Epitop, ausgewählt aus der Gruppe der Epitope ß1 bis ß9 (besonders bevorzugt ß2 bis ß8) der ßhCG-Untereinheit der Epitope cfß1 bis cfß13 auf dem ßhCG-core Fragment und der konformationsabhängigen Epitope αß1 bis αß4 des intakten αß-Heterodimers der Klassifizierung der "International Society of Oncodevelopmental Biology and Medecine" (ISOBM) (18-20) . Bezogen werden können derartige Antikörpern beispielsweise von der Firma BIOTREND Chemikalien GmbH, Köln, Deutschland oder Serotec Düsseldorf, Deutschland.

Als Standard für eßhCG bzw. als Negativkontrolle für tßhCG sind in dem Testkit bevorzugt eßhCG, oder Peptide mit einer Aminosäuresequenz aus sechs bis 15 Aminosäuren aus dem im Bereich der Aminosänreposition 2 und 4 bzw. im Bereich der Aminosäureposition der 117 der Sequenz des eßhCG (SEQ ID No 7), d. h der Eiptope eß1 bzw, eß9, vorzugsweise Peptide der SEQ ID No. 1, 12, 3, 14 bzw. deren Teilsequenzen deren Lösungen enthalten.

Als Standard für tßhCG bzw. als Negativkontrolle für eßhCG sind in dem Testkit bevorzugt tßhCG, oder Peptide mit einer Aminosäuresequenz aus sechs- bis 15 Aminosäuren aus dem im Bereich der Aminosäureposition 2 und 4 oder im Bereich der Aminosäureposition der 117 der Sequenz des tßhCG (SEQ ID No 8),, d. h der Biptope ß1 bzw. ß9, vorzugsweise Peptide der SEQ ID No. 2, 13, 4, 15 bzw. deren Teilsequenzen deren Lösungen enthalten.

Ein weiterer Gegenstand der Erfindung ist die Verwendung der endometriale ß-Untereinheit (eßhCG) von humanen Choriongonadotropin (eßhCG) mit der Aminosäuresequenz gemäß SEQ ID No. 10 oder der Gensequenz für eßhCG gemäß SEQ ID No 7 als Marker für die Schwangerschaftsdiagnose oder zur Diagnose der Aufnahmebereitschaft der Uterusschleimhaut für eine befruchtete Eizelle. Gegenstand der Erfindung sind auch die isolierten Peptidsequenzen gemäß SEQ ID No. 1,3 12 und 14.

Die Erfindung umfasst auch die Verwendung der erfindungsgemäßen eßhCG spezifischen Antikörper, der isolierten Peptidsequenzen gemäß SBQ ID No. 3 bzw. 14 und des erfindungsgemäßen. Testkits zur Schwangetschaftsdiagnose oder zur Diagnose der Aufnahmebereitschaft der Uterusschleimhaut für eine befruchtete Eizelle.

Die Erfindung wird anhand der nachfolgenden Ausführungsbeispiele näher erläutert, ohne auf diese beschränkt zu sein:
Ausführungsbeispiel 1: Gewinnung eines polyklonalen Antikörpers spezifisch für das epitheliale endometriale hCG-Molekül (eβhCG) zum βhCG-Epitop β9 am C-terminalen Ende (AS 109-123).
Ausführungsbeispiel 2: Gewinnung eines Antikörpers spezifisch für das epitheliale endometriale hCG-Molekül (eβhCG) zum βhCG-Epitop β1 (AS 1-15).
Ausführungsbeispiel 3: Generierung von monoklonalen Antikörpern.
Ausführungsbeispiel 4: Verfahren zum Nachweis des eβhCG in Körperflüssigkeiten und Gewebehomogenaten mittels ELISA.
Ausführungsbeispiel 5: Zusammensetzung eines Testkists zum Nachweis des eβhCG in Körperflüssigkeiten und Gewebehomogenaten mittels ELISA.
Ausführungsbeispiel 6: Verfahren zur Feststellung optimaler Implantationsbedingungen durch die Bestimmung von endometrialem hCG.
Ausführungsbeispiel 7: Verfahren zum Feststellen von physiologischen Endometriumszuständen und zur Erfassung von Fertilisationsstörungen im menstruellen Zyklus durch die Bestimmung von ehCG.
Ausführungsbeispiel 8: Verfahren zur retrograden Beurteilung optimaler Implantationsbedingungen durch die Bestimmung von endometrialem hCG im Menstrualblut.
Ausführungsbeispiel 9: Verfahren zur Diagnostik für eine Differenzierung zwischen mütterlich-dezidualen vs. embryonal trophoblastären Störungen bei Abortneigung und beginnendem Abort.
Ausführungsbeispiel 10: Verfahren zum Frühgebwisscreening bzw. zur Diagnostik des Geburtsbeginnes.

### Ausführungsbeispiel 1:

Für die Gewinnung von Antikörpern, die spezifisch das endometrial und dezidual translatierte eßhCG gemäß SEQ ID No 10 erkennen, wird in diesem Ausführungsbeispiel das folgende Dekapeptid (SEQ ID No. 12) als Antigen mit hoher Antigenizität aus dem für die Antikörpergewinnung zum eβhCG-Epitop eβ9 in der Erfindung empfohlenen Aminosäuresequenzbereich (SEQ ID No. 1) verwendet:
P1: Cys-Asp-Asp-Pro-Arg-Phe-Gln-Ala-Ser-Ser (SEQ ID No. 12)

Dieses P 1 ist ein synthetisches Peptid mit 10 Aminosäuren (AS) aus der Aminosäuresequenz 109 - 123 des Exon 3 der endometrialen Variante des β7- und β6-Gen. Dieses Peptid unterscheidet sich vom bekannten, dem C-terminalen Peptid (CTP-βhCG) nahen Epitop β9 der trophoblastären βhCG-Untereinheit durch ein Alanin (Ala) - statt Aspartat (Asp) - an Position 8 des Peptids (Aminosäureposition 117 im βhCG). Die ausgewählte Aminosäuresequenz unterscheidet sich beträchtlich von der Sequenz der βLH-Untereinheit (SEQ ID No. 11). Alternativ können auch andere Peptide mit 7 bis 15 Aminosäuren aus dem Sequenzbereich aus dem Aminosäuresequenzbereich eβhCG-Epitop eβ9 (SEQ ID No. 1) verwendet werden, welche ein Alanin (Ala) an der Position aufweisen, die der Aminosäureposition 117 im eβhCG (SEQ ID No 10) entspricht.

Zur Gewinnung von Kontroll-Antikörpern, die spezifisch die β5, β8, β3 - Untereinheiten des bekannten trophoblastären hCG (thCG) (SEQ ID No 8) erkennen, wird in diesem Ausführungsbeispiel das folgende synthetische Dekapeptid (SEQ ID No 13) als Antigen mit hoher Antigenizität aus dem für die Antikörpergewinnung zum βhCG-Epitop β9 in der Erfindung empfohlenen Aminosäuresequenzbereich (SEQ ID No. 2) im Epitop β9 nahe dem CTP-βhCG eingesetzt:
K1: Cys - Asp - Asp - Pro - Arg - Phe - Gln - Asp - Ser - Ser (SEQ ID No. 13)

Die Peptide P1 und K1 wurden durch konventionelle Solid-phase-Peptidsynthese hergestellt, durch Gelfiltration und Ionenaustauschchromatographie gereinigt und durch HPLC spezifiziert (23, 49).

Die aus dem in der Erfindung empfohlenen Aminosäuresequenzbereich für das βhCG-Epitop β9 in diesem Ausführungsbeispiel ausgewählten. Peptide P1 und K1 werden mit dem BZ Antibody Production and Purification Kit, Carboxyle reactive (Pierce Chemical Co., Rockford, IL) nach der Herstellevorschrift an das Protein keyhole limpet hemocyanin (KLH) als Carrier gebunden (53). Nach der entsprechenden Vorschrift wurden die Peptide für den nachfolgend beschriebenen ELISA an Bovine Serumalbumin (BSA) als Carrier gebunden.

Für die Herstellung polyklonaler Antikörper werden fünf 12 Wochen alten Kaninchen (New Zealand White, jeweils circa 2 kg im Gewicht) verschiedene Lösungen mit einem Gesamtvolumen von jeweils 500 µl i. p. injiziert. Kaninchen #1 erhält 200 µg KLH-gebundenes Peptid P1 in 0,1% NaCl mit 1:1 Adjuvanz Specol (ID-DLO, Lelystad), Kaninchen #2 erhält 500 µg KLH-gebundenes Peptid K1 in 0,1% NaCl mit 1:1 Adjuvanz Specol, Kaninchen. #3 ersielt nur 0,1% NaCl mit 1: 1 Adjuvanz Specol. 14 Tage später wird die Injektion mit jeweils der gleichen Lösung i. p. wiederholt (Boosterung). Eine letzte Boosterung erfolgt 3 Wochen nach der ersten Boosterung mit der gleichen Lösung. 14 Tage nach der letzten Boosterung werden Seren entnommen. Die Seren werden in einem ELISA nach Standardbedingungen auf das Vorhandensein spezifischer Antikörper gegen P1 untersucht.

Für den ELISA wurde das BSA-gebundene Peptid P1 zunächst in einer Konzentration von 10 µg/ml in einem Beschichtungspuffer (0,1 mol/Liter Natriumcarbonat/Bicarbonat, pH 9,6) in 50 µl/Vertiefung auf eine MaxiSorp ELISA-Platte (Nunc) aufgebracht und für 1 h bei 37 °C inkubiert. Parallel dazu wurde eine Platte (=Negativ-Kontrollplatte) entsprechend mit einer Lösung des BSA-gebundenen Kontrollpeptids K1 beschichtet. Die Vertiefungen wurden anschließend fünfmal mit Phosphat-gepufferter Kochsalzlösung mit Zusatz von 0,1% Tween 20 (PBS-T) gewaschen. Dann wurden jeweils 200 µl einer 10%-eigen Milchpulver-Lösung in PBS-T zugegeben und für 1 h bei 37°C inkubiert und einmal mit PBS-T gewaschen. Die so vorbereiteten Platten wurden mit den Immunseren inkubiert, 3 x mit PBS-T gewaschen und 0,5 h mit einem biotinylierten Anti-Kaninchen~IgG (Dako) als sekundärem Antikörper inkubiert und dreimal mit PBS-T gewaschen.

Anschließend wurde eine 1:200U Verdünnung eines Streptavidin-Peroxidase-Konjugats (Sigma) in PBS-T zugegeben. Nach 30minütiger Inkubation bei 37 °C wurde die Platte zweimal mit PBS-T und einmal mit PBS gewaschen und ein Substrat (100 µl), das o-Phenyldiaminhyhrochlorid enthielt, zugegeben. Die gelbbraune Farbentwicklung wurde nach 5 Minuten durch Zugabe von 50 µl 2M H₂SO₄ gestoppt und die optische Dichte wurde bei einer Wellenlänge von 490 nm (Referenz-Wellenlänge: 650 nm) bestimmt.

Hierbei konnten P1 -spezifische Antikörper nur bei Kaninchen #1 bei den Seren, die ab dem 7. Tag nach Injektion gewonnen worden waren, nicht jedoch bei dem Seren vor der Injektion sowie bei keinem Sesam von Kaninchen #2 und #3 detektiert werden. Diese Versuche demonstrieren, dass das Peptid P1 in der Lage ist, spezifische Antikörper in Kaninchen zu induzieren.

Zur immunaffinitäts chromatographischen Aufreinigung der Antikörper aus dem Serum wird das Peptid P1 entsprechend der Herstellervorschrift des EZ^{™} Antibody Production and Purification Kits, Carboxyl reaktive (Pierce) an eine Diaminodipropylamine-Säule immobilisiert (53). Das Serum, aus dem die spezifischen Antikörper gereinigt werden sollten, wurde zur Inaktivierung fur 30 Minuten bei 56 °C inkubiert. Danach wurde es auf die Säule gegeben und lief hindurch. Anschließend wurde die Säule mit 20 ml PBS und mit 10 ml einer 0.5 Mol/Liter MgCl₂-Lösung gewaschen. Die Elution der spezifisch gebundenen Antikörper erfolgte durch Zugabe von 3 ml 3 Mol/Liter MgCl₂, gefolgt von 3 ml 4 Mol/Liter MgCl₂. Die Eluate wurden in Dialyse-Schläuche (Pierce) gegeben und über Nacht bei 4 °C gegen 1 Liter PBS dialysiert. Anschließend wurde der Proteingehalt der dialysierten Präparationen mittels des BCA-Protein Kits (Pierce) bestimmt, die Reinheit durch SDS-PAGE und Coomassieblue-Färbung sowie die Spezifität der Antikörper-Präparationen mittels ELISA nachgewiesen.

### Ausführungsbeispiel 2:

Auch für die weiteren Aminosäuredifferenzen zwischen tβhCG und eβhCG im βhCG-Epitopabschnitt β1 in den Aminosäurepositionen +2 (Lys zu Arg) und +4 (Pro zu Met) wird ein eβhCG-spezifischer Antikörper und ein tβhCG-spezifischer Kontroll-Antikörper hergestellt.

Zur Generierung von Antikörpern, die spezifisch das endometrial und dezidual translatierte eβhCG gemäß SEQ ID No 10 erkennen, wird in diesem Ausführungsbeispiel das folgende synthetische Peptid (SEQ ID No. 14) als Antigen mit hoher Antigenizität aus dem für die Antikörpergewinnung zum βhCO-Epitop eβ1 in der Erfindung empfohlenen Aminosauresequenzbereich (SEQ ID No. 3) verwendet:
P2: Ser-Arg-Glu-Met-Leu-Arg-Pro-Arg-Cys-Arg-Pro (SEQ ID No. 14)

Dieses P2 ist ein synthetisches Peptid aus dem Aminosäuresequenzbereich 1-15 im Exon 2, das sich vom bekannten Epitop β1 der trophoblastären βhCG-Untereinheit in zwei Aminosäurepositionen unterscheidet. Die ausgewählte Aminosäuresequenz P2 unterscheidet sich in diesem Epitop-Abschnitt auch beträchtlich von der Sequenz der βLH-Untereinheit (SEQ ID No. 11). Alternativ können auch andere Peptide mit 7 bis 15 Aminosäuren aus dem Sequenzbereich aus dem Aminosäuresequenzbereich eβhCG-Epitop eβ1 (SEQ ID No. 3) verwendet werden, welche ein Argenin (Arg) und ein metheonin (Met) an den Positionen aufweisen, die den Aminosäurepositionen 2 und 4 im eβhCG (SEQ ID No 10) entsprechen.

Zur Gewinnung von Kontroll-Antikörpern, die spezifische tβhCG (β5, β8, β3) erkennen, wird entsprechend mit dem adäquaten, als Beispiel ausgewähltes synthetisches Peptid (SBQ ID No. 15) der Aminosäuresequenz des βhCG-Gens β5 aus dem Epitop ß1 (SBQ ID No. 4) - Aminosäuresequenzbereich AS 1 bis 15 des tßhCG (SEQ ID No 8) verfahren:
K2: Ser- Lys - Glu - Pro - Leu - Arg - Pro - Arg - Cys - Arg - Pro (SEQ ID No. 15)

Die Immunisierung der Kaninchen war mit P2 und K2 genauso erfolgreich, wie mit P1 und K1. Es wurden mit P2 ebenfalls eβhCG-spezifische Antikörper erhalten, die keine Cross-Reaktivität für K2 oder tβhCG und auch keine Crossreaktivität zu βLH zeigten.

### Ausführungsbeispiel 3:

Die Generierung von monoklonalen Antikörpern (mAb) erfolgt entsprechend der in der Literatur gut beschriebenen Hybridomherstellung für die ISOBM-MAb h54, 264, 277, 278, 287, 282 und 313 für Epitop β8, FB-12 und 280 für Epitop β9 und 265, 274 und 284 für Epitop β1 mit Carrier-gebundenen synthetischen ßhCG-Peptidsequenzen (18, 20-24, 45-48), wobei in den Peptidsequenzen die Aminosäuren an Position +2 , +4 und +117 für das endometriale hCG (eßhCG) ausgetauscht wurden. Die Herstellung der ISOBM-mAb hatte gezeigt, dass die Herstellung von Hybridomen, welche Antikörper der gewünschten ßhCG-Spezifität sezernieren, zuverlässig wiederholbar ist (18, 24, 45-48).

Zur Hybridomherstellung wurden dabei die Carrier-gebundenen synthetischen eβhCGspezifischen Peptidsequenzen P1 und P2 (SEQ No. 12 und 14) sowie die Kontrollpeptide K1 und K2 (SEQ ID No. 13 und 15) verwendet.

Die Herstellung der monoklonalen Antikörper für P1 wird nachfolgend näher erläutert:

Die Immunisierung erfolgte in BALB/c-Mäusen. Dazu werden pro Maus etwa 1 mg gereinigtes Carrier-gebundenes Peptid benötigt.

Immunisierung: 8 sechs bis acht Wochen alte weibliche BALB/c-Mäuse (Roche, Institut für Biologisch-Medizinische Forschung, Basel, Schweiz) werden mit dem entsprechend Ausführungsbeispiel 1 hergestellten KLH-gebundenen Peptid P1 für jedes Tier nach folgendem Protokoll immunisiert (18, 20, 24, 45-51): Die erste Immunisierung erfolgte durch subkutane Injektion von 50-150 µg βhCG-Peptidimmunogens am Carrier pro Tier in kompletter Freund'scher Adjuvanz. Die weiteren Immunisierungen erfolgen jeden zweiten Tag durch Injektion derselben Menge Immunogen in inkompletter Freund'scher Adjuvanz. Am Tag 17 erhalten die Mäuse eine intraperitonale Immunisierung mit wiederum je 50-150 µg des Antigens in PBS für jedes Tier. Die Immunseren werden mit dem hCG POD-System auf freigesetzte Antikörper getestet (ELISA in Ausführungsbeispiel 1). Die Mäuse mit den hohen hCG-Antikörper-Immunantworten (etwa 3) werden mit nochmals 50-150 µg βhCG-Immunogen geboostert und nach drei Tagen für die Fusion vorgesehen.

Fusion: Nach der Immunisierung werden aus den Kniegelenk-Lymphknoten und Milzen derart immunisierter Mäuse die Splenozyten (B-Lymphozyten) isoliert und mit Zellen der Mausmyeloma-Zelllinie P3-X63-Ag8.653 (American Type Culture Collection) nach der Methode von Köhler und Milestein (51), wie bei Kovalevskaja (47) beschrieben, fusioniert. Das Verhältnis von Splenozyten zu Myelomazellen beträgt dabei 4:1 bis 6:1. RPMI-1640 mit 10 % fetalem Kälberserum (FKS) oder Polyethylenglycol 1500 (Sigma) wird als Fusionsmedium verwendet. Das Immunserum der ausgewählten Mäuse wird als Positivkontrolle gesammelt.

Selektion der fusionierten Zellen (Hybridomazellen): Die gebildeten Hybridomazellen werden nach der Fusion von den nicht fusionierten Myelomzellen abgetrennt, in Mikrotiterplatten verteilt und zusammen mit peritonealen Asciteszellen der Maus für eine Woche in einem RPMI-Kulturmedium, das Hypoxanthin, Aminopterin und Thymidin (HAT) mit 10% FKS enthält, kultiviert (46-50). Die Hälfte des Mediums wurde aller drei Tage ersetzt. An den Tagen 12-14 nach Fusion wird ein Anteil des Kulturüberstandes von den Wells auf die Anwesenheit von hCG-Antikörpern in einem ELISA geprüft (Screening der Oligoklone).

Das Screening der Oligoklone erfolgt in einem ELISA wie in Ausführungsbeispiel 1 beschrieben, mit dem Unterschied, dass Anstelle des biotinylierten Anti-Kaninchen-IgG- ein biotinylierter Anti-Maus-IgG-Antikörper (Dako) eingesetzt wurde.

Die Zellüberstände von 10 % der gescreenten Wells zeigten mit der ELISA-Platte, auf der P1 immobilisiert war - nicht jedoch mit der Negativ-Kontrollplatte - eine Farbreaktion im ELISA. 10 % der erhalten Oligo-Klone erkennen daher spezifisch das Peptid P1 - und nicht K1.

Drei besonders produktive Ig-positive Zellklone, P1.1, P1.2 und P1.3 zeigten einen besonders hohen Antikörpertiter mit der gewünschten ehCG-Spezifität und wurden für die weitere Subklonierung ausgewählt.

Subklonierung : Die selektierten Oligoklone werden jetzt zur Monoklonalität subkloniert (50). Dafür wurden positive Klone selektiert und *in vitro* weiter vermehrt (cloning by limiting dilution method). Die isolierten Kolonien wurden nochmals mit ELISA getestet. Die positiv getesteten Klone werden für den nächsten Zyklus der Klonierung eingesetzt. Drei Zyklen der Klonierung sind erforderlich, um spezifische, stabile Klone zu erhalten. Sie wurden für die Bildung von 100 ml Überstand mit dem manoklonalen Antikörper verwendet.

Die molekularen hCG-Antikörper wurden anschließend durch Affinitätschromatographie mit dem Protein A-Sepharose-Purification-System für monoklonale Antikörper (Biorad) gereinigt. Die Reinheit des mAb wurde durch SDS-Polyacrylamid-Gelelektrophorese geprüft und anschließend die Proteinkonzentration bestimmt (18, 52).

Die Spezifität des Antikörpers wurde sowohl gegen das heterodimere αβhCGF-Molekül mit eßhCG als β-Untereinheit, als auch gegen das angegebene Peptid P1 im oben beschriebenen ELISA nachgewiesen.

Die derart durch Immunisierung, Isolierung, Hybridisierung und Feinreinigung hergestellten monoklonalen Antikörper werden bei - 20° C gelagert.

Die Herstellung von monoklonalen Antikörpern mit dem Peptid P2 erfolgte nach der gleichen Vorschrift und führte zu vergleichbaren Ergebnissen. Es wurden mit P2 ebenfalls eβhCGspezifische Antikörper erhalten, die keine Cross-Reaktivität für K2 oder tβhCG zeigten.

### Ausführungsbeispiel 4:

Zum Nachweis des epithelialen endometrialen und dezidualen hCG (ehCG) in Körperflüssigkeiten und Gewebehomogenaten werden die nach Ausführungsbeispiel 1 bis 3 gewonnenen βhCG Epitop β8- und βhCG Epitop β1 -spezifischen endometrialen respektive dezidualen βhCG-Antikörper an Mikrotiterplatten adsorbiert und Testsysteme auf der Basis der ELISA-Technik entwickelt. Als Kontrollsystem dienen adäquate ELISA-Anordnungen unter Verwendung der vergleichbaren trophoblastären βhCG (thCG)-Antikörper des jeweiligen βhCG-Epitopes β1 und β8 (18, 21-24, 47).

*Sandwich-ELISA:* Entsprechend Ausführungsbeispiel 3 werden die immungereinigten P1 und P2 (eßhCG) sowie K1 und K2 (tβhCG) spezifischen monoklonalen eßhCG-Antiköper als Etstantikörper in einer Lösung von 100µl/well an MaxiSorp ELISA-Platten (Nunc, 96 wells) adsorbiert (10 µg/ml in 200 mM Bicarbonat-Puffer, pH 9,6, 1 Stunde bei 37° C oder über Nacht, 4° C). Die wells werden anschließend zweimal mit Waschpuffer (10 mM PBS, pH 7,2 mit 0,05% Tween 20) gewaschen und eine Stunde mit Blockingpuffer (1 % BSA in PBS pH 7,2) inkubiert.

Es folgt die Inkubation (jeweils 100 µl, 1 Stunde , 37° C) mit dem Serum, in dem das endometriale hCG (eβhCG) nachgewiesen werden soll. Das Serum wird dazu 1:10 bis 1:1000 in Blocking-Puffer verdünnt. Als Standardreihe der hCG-Bestimmung werden zusätzlich die synthetischen Peptide P1, P2 (endometriumspezifisch) und K1 und K2 (trophoblastspezifisch) in sechs verschiedenen Konzentrationsstufen zwischen 0 und 1000 ng/ml inhibiert.

Wenn hCG in den verwendeten Proben anwesend ist, wird es sich an den immobilisierten endometrium- oder trophoblastspezifischen Antikörper der wells binden. Das Assaysystem benutzt nach jeweiligen Waschschritten den zweiten biotinylierten anti-βhCG-Antikörper, der sich als Sandwich an den immobilisierten solid phase-βhCCr-Antikörper-/βhCG-Komplex bindet. Als zweiter monoklonaler hCG/βhCG-Antikörper der sowohl das endometriale als auch trophoblastäre Gesamtmoleküll hCG und seine β-Untereinheit erkennt wird in diesem Ausführungsbeispiel ein biotinylierter Antikörper spezifisch für das hCG β2 Epitop (INN-22 Serotec) verwendet. Nach Inkubation bei Zimmertemperatur und weiteren Waschschritten zur Entfernung des überschüssigen enzymgebundenen βhCG-Antikörpers wird fortgefahren wie im Ausführungsbeispiel 1 dargestellt. Die Detektion wird eingeleitet mit einer 1:2000 Verdünnung des streptavidin-Peroxydase-Konjugates (Sigma) in PBS-T.

Nach 30 minütiger Inkubatin bei 37° C wurde die Platte zweimal mit PBS-T und einmal mit PBS gewaschen und ein Substrat (100 ul), das o-Phenylendiammhydrochlorid enthielt, zugegeben. Die gelbbraune Farbentwickling wurde nach 5 Minuten durch Zugabe von 50 µl 2 M Schwefelsäure gestoppt und die optische Dichte wurde bei einer Wellenlänge von 490 nm (referenzwellenlänge: 650 nm) bestimmt.

### Ausführungsbeispiel 5:

Ein Testkit zum spezifischen Nachweis des endometrialen respektive dezidualen hCG und seiner βhCG-Untereinheit (eßhCG) in Körperflüssigkeiten und Gewebehomogenaten in einem ELISA enthält beispielsweise folgende Komponenten:
1. Eine mit eßhCG-spezifischen Antikörper Klon P12 aus Ausführungsbeispiel 3 (spezifisch für im Endometrium und Dezidua exprimiertes eßhCG, erkennt nicht tβhCG) vorbeschichtete ELISA-Platte (jeweils 10 µg pro well),
2. Sechs Verdünnungen des Peptids P1 als Standardreihe (0, 10, 50 100, 500, 1000 µg/ml),
3. Waschpuffer PBS-T (10 mM PBS, pH 7,2 mit 0,05% Tween 20),
4. Blockingpuffer (1 % BSA in PBS pH 7,2),
5. biotinylierter GesamihCG/βhCG-Antiköxper als zweiter hCG-Antikörper spezifisch Für das hCG β2 Epitop (INN-22 Serotec),
6. Streptavidin-HRP-Konjugat (Dako),
7. PBS (Dako),
8. o-Phenylendiamin als Substrat,
9. 2 M Schwefelsäure als Stoplösung.

Alternativ zu Komponente 5 enthält der Testxit beispielsweise als zweiten hCG-Antikörper einen biotinylierten Antikörper spezifisch für das hCG β4 Epitop (INN-24, Serotec).

Bin adäquater Testkit als Kontroll-Kit oder zur spezifischen Bestimmung des trophoblastären hCG in Körperflüssigkeiten oder Gewebehomogenaten enthält beispielsweise die obigen Komponenten 3. bis 9. und anstelle Komponente 1. einen durch die Immunisierung mit K1 erhaltenen tβhCG-spezifischen Antikörper und anstelle Komponente 2. entsprechende Verdünnungen des Kontrollpeptides K1 als Standardreihe.

Mit dem Kit zum spezifischen Nachweis des eßhCG kann die Quantifizierung und Bewertung der spezifischen epithelialen endomeirialen hCG-Sekretion (ehCG) in Körperflüssigkeiten und Zell- oder Gewebehomogenaten der frühen bis mittleren Sekretionsphase des mentruellen Zyklus optimale imptantationsbedingungen (Ausführungsbeispiel 6) wie auch mögliche Fertilisations- störungen (Ausführungsbeispiel 7) sowohl prospektiv als auch retrograd unter dem Aspekt der endometrialen Diagnostik und Therapiekontrolle erfaBt werden.

### Ausführungsbeispiel 6:

Für die prospektive Diagnostik der Embryorezeptivität in der frühen Sekretionsphase eines aktuellen Zyklus werden von der Patientin z. B. mit Kinderwunsch mittzyklisch ein Abstrich aus dem Zervicalkanal mit Zervikalsekret oder ein Scheidenabstrich mit Vaginalsekret für die diagnostische Bewertung der Implantationsbedingungen vorgenommen. Dieser Abstrich wird auf die vorhandene beginnende oder in Gang befindliche Exprimierung bzw. Sekretion des eßhCG mittels dem in Ausfühungsbeispiel 4 beschriebenen ELISA untersucht Damit können Aussagen über die Qualität der sekretorischen Transformation und der zu erwartenden und Rezeptivität des Endometriums getroffen werden.

Im Rahmen der in vitro-Fertilisation wird zwei Tage nach der Follikelpunktion kurzfristig ein Wattebausch in die Zervix eingelegt oder ein Scheidenabstrich abgenommen und mittels dem in Ausführungsbeispiel 4 beschriebenen ELISA die Aktivierung des eßhCG diagnostiziert. Ein positiver eßhCG-Nachweis signalisiert ein rezeptives EnLdomeinum, und der noch in der Kultur befindliche Embryo kann 1 bis 2 Tage später transferiert werden. Fällt hingegen der Test negativ aus, wird der Embryo kryokonserviert und im nächsten für eßhCG positiv befundeten Zyklus in die Gebärmutterhöhle eingespült. Es können damit Entscheidungen zum Embryotransfer oder zur Insemination der hormonell stimulierten Patientin für den aktuellen oder erst nächstfolgenden Zyklus getroffen werden. Neben dem Cervixsekret kann aber auch Probenmaterial (Gewebe, Zellen, Perfust) anderer epithelialer Organe wie Mundschleimhaut oder Vaginalschleimhaut eingesetzt werden. Da alle epithelialen Organe dem Zyklus mehr oder wenig unterworfen sind, können auch diese in die Untersuchung einbezogen werden.

### Ausführungsbeispiel 7:

Mit diesem Ausführungsbeispiel können bei Patientinnen physiologische oder pathalogische Endornetriumzustände detektiert und mögliche Fertilisationsstörungen unter dem Aspekt der endometrialen hCG-Diagnostik und Therapiekontrolle in aktuellen Zyklus oder Folgezyklus erfaßt und bewertet werden.

Zu diesem Zweck werden der Patientin in der sekretorischen Phase der endometrialen Transformation, vor allem der mittleren Sekretionsphase um den 20.-24. Zyklustag, ein Abstrich aus dem Zervikalkanal mit Cervikalsekret oder Scheidenabstrich mit Vaginalsekret für die diagnostische Bewertung vorgenommen.

Dieser Abstrich wird auf die vorhandene beginnende oder in Gang befindliche Exprimierung bzw. Sekretion des eßhCG mittels dem in Ausführungsbeispiel 4 beschriebenen BUS untersucht. Damit können Aussagen zur fehlenden, unterwertigen oder hohen sekretorischen Transformation des Endometeriums und der Qualität der zu erwartenden Rezeptivität des Endometriums für die Patientin getroffen werden.

Das Vorhandensein von eindeutig meßbarem eßhCG am 20. bis 24. Zyklustag signalisiert ein gesundes zeit und funktionsgerecht umgewandeltes Endometrium. Dies ist gleichzeitig Ausdruck einer ungestörten Interaktion zwischen Hypothalamus und Hypophyse, dem Eierstock und dem Uterus. Mit dem angegebenen Verfahren des Abstriches von Patientinnen für Untersuchungen des ehCG in Körperflüssigkeiten und Zell- und Gewebehomogenaten kann die Diagnostik und Therapiekontrolle der Uterusfunktion vorgenommen werden. Für diese ehCC-Bestimmungen mit den obengenannten Methoden kann auch das kürettierte Endomefrium von Strichabrasiones nach diagnostischer Indikation eingesetzt werden,

### Ausführungsbeispiel 8:

Auch die Abnahme von Menstrualblut bei Patientinnen nach unstimulierten, stimulierten oder gestörten Zyklus stellt eine wichtige, einfache und bisher nicht genutzte Methode der retrograden Implantationsdiagnostik dar, um Aussagen über die sekretorische Transformation des Endometriums des vorangegangenen Zyklus zu detektieren und um gegebenfalls Aussagen für die Rezeptivität des Folgezyklus zu treffen. Diese nicht invasive Methode kann die invasive Methode der diagnostischen Strichabrasio in ihrer Aussage ergänzen oder ersetzen.

Das Menstrualblut im Ergebnis der endometrialen Desquamation wird wie Peripherblut nach Zentrifugation zur Abtrennung von Zellmaterial und Stroma für die direkten Messungen der endometnalen hCG-Sekretion (ehCG) mit spezifischen ehCG-Antikörpern im BUS-TEST entsprechend Ausführungsbeispiel 4 eingesetzt. Das Menstrualblut wird nach spontanem Zyklus, nach Hormontherapie, nach IVF und BT ohne erfolgreiche Implantation sowie bei vorgesehener Diagnostik des Zyklus bei Kinderwunschpatientinnen und bei IUD-Patientinnen, Myom und Endometriose gewonnen. Dabei erfolgte eine parallele Analyse von Peripherblut zum selben Zeitpunkt zum Ansschluß eines ebenfalls erhöhten Serumwertes von ehCG.

Das vom Menstrualblut abgetrennte epitheliale und stromale Zellmaterial des endometrialen Gewebes nach Desquamation wird ebenso wie das Menstrualptasma mittel ELISA-Test zur Bewertung der Expression und Sekretion des endometrialen hCG im vorangegangenen Zyklus genutzt

### Ausführungsbeispiel 9:

Die Diagnostik für eine Differenzierung zwischen mütterlich-dezidualen vs. embryonal-trophoblastären Störungen bei Abortneigung und beginnendem Abort basiert darauf, dass im mütterlich-dezidualem Gewebe der Schwangerschaft wie im sekretorischen Endometrium eßhCG exptimiert wird. Im embryonal-trophoblastären Gewebe der Plazentta wird frophoblastäres hCG exprimiert und translatiert. Die hCG-Konzentrationen des Peripherblutes in der Schwangerschaft zeigen ein Sekretionsmaximum im ersten Trimenon und sind über das zweite und dritte Trimenon beträchtlich.

Mit diesem Ausführungsbeispiel wird eine Anwendung aufgezeigt, im Peripherblut, aber auch im Vaginal- oder Zervixsekret nach Abstrich, im freigesetzten Fruchtwasser bei perforierter oder geplatzter Base, im Lochilalblut sowie anderen epithelialen Sekreten und/oder deren Zell- und Gewebehomagenatea von Patientinnen in der Schwangerschaft hCG differenziert als endometrial/deziduales hCG (chCG) und trophablastäres hCG (thCG) im ELISA-Test oder in der quantitativen Real time RT-PCR zu bestimmen.

Bei drohendem Abort, gekennzeichnet durch eine beginnende uterine Blutung, wird therapeutisch polypragmatisch vorgegangen, weil zumeist die Ursache der Störung unklar ist Bei drohendem Abort kann aber durch eine Differenzierung von embryonalen hCG (ehCG) und dem trophoblastären hCG (thCG) zwischen einer mutterlich-dezidualen vs. trophoblastärembryonalen Ursache der Störung unterschieden werden.

Dazu wird Serum von der Patientin oder die oben angegebenen weiteren Körperflüssigkeiten und Zell- und Gewebehomogenate entnommen und mit den beiden in der Erfindung entwickelten BUSA-Kits auf ehCG und/oder thCG untersucht (Ausführungsbeispiel 4). thCG kann auch mit herkömmlichen kommerziellen Kits erfaßt werden, die für übliche plazentare hCG-Messungen in der Schwangerschaft empfohlen werden. (DPI, Abbott, Serono, Roche, Baxter). Ein niedriges ehCG zeigt eine beginnende deziduale Insuffizienz, während ein im verhältnis niedriges thCG eine Störung der fetoplazentaren Einheit signalisiert Während die erste Störung behandelbar ist, muss bei der Störung der thCG-Sekretion überprüft werden, ob eine Therapie machbar und sinnvoll ist.

Auch läßt sich die Prognose einer drohenden Fehlgeburt aus dem Blut detektieren. Dazu wird Abortblut mit einem Spekulum aus dem hinteren Scheidengewölbe entnommen und eine Bestimmung des ehCG mit dem in der Erfindung entwickelten ELISA-Kit vorgenommen. Bei geringen Blutungen kann mit einem Wattebausch Blut oder blutiger Cervixschleim entnommen und mittels ELISA Test (Ausführungsbeispiel 4) und/oder quantitativer Real time RT-PCR auf eßhCG untersucht werden. Ein hoher ehCG Nachweis weist auf eine dezidnale Störung hin. Bei sehr hohen ehCG-Werten muss mit einer nicht therapierbaren Fehlgeburt gerechnet werden.

Das Verfahren dieses Ausführungsbeispiels kann auch zur Therapiekontrolle in der Behandlung dezidualer Störungen genutzt werden.

### Ausführungsbeispiel 10:

Das Frühgeburtssereening bzw. die Diagnostik des Geburtsbeginnes basiert darauf, dass das embryonale hCG (ehCG) im Genitaltraktes vor der Frühgeburt erhöht ist, während das ehCG im Serum erniedrigt gefunden wird Aus diesem Grund läßt sich das obengenannte Verfahren zum Frühgeburtsscreening einsetzen. Bin gleichsinniges Muster wird auch bei Geburtsbeginn am Ende der Schwangerschaft gefunden.

Bei Patientinnen werden Für ein Frühgeburtsscreening oder zur Diagnose des Geburtsbeginns aus dem Genitaltrakt (Cervix, hinteres Scheidengewölbe) Sekret oder Zellen entnommen. Im Sekret wird mit obengenannten ELISA- Test das ehCG bestimmt, während bei der Gewinnung von Zellen im Sekret mittels der quantitative Real time RT-PCR die ehCG β7-Expression erfaßt wird. Ein erhöhter ehCG-Nachweis im Sekret signalisiert eine beginnende Frühgeburt, ebenso wie ein verminderter ehCG β7 in den Zellen des Genitaltraktes.

Außerdem ist ein Frühgeburtenscreening durch eine Serumuntersuchung möglich. Dabei wird Serum von der Patientin gewonnen und das ehCG bestimmt. Bei niedrigen bzw. abfallenden ehCG-Werten ist mit einer Frühgeburt zu rechnen.

Anstelle des in den Ausfuhrungsbeispielen beschriebenen ELlSA-Tests zum Nachweis der eßhCG Konzentration kann in den Gewebeproben auch eine quantitative Erfassung der ehCG-Genexpression durch Real time RT-PCR erfolgen.

### Abkürzungsverzeichnis:

In der Erfindungsbeschreibung werden folgende Abkürzungen verwendet:
- αhCG: alpha-Untereinheit des humanen Choriongonadotropin
- βhCG: beta-Untereinheit des humanen Choriongonadotropin
- BSA: Bovines Serumalbumin
- CTP: C-terminalesPeptid
- EDTA: Ethylendiamintetraacetat
- ET: Embryotransfer
- eßhcG: endometriale beta-Untereinheit des humanen Chorionganadotropin
- ehCG: endometriales humanes Choriongonadotropin (eßhCG + αhCG)
- hCG: Humanes Choriongonadotropin
- IgG: Immunglobulin Gamma
- ISOBM: International Society of Oncodevelopmental Biology and Medicine
- IUD: Intrauterine Device
- IVF: In-vitro-Fertuisatton
- KLH: keyhole limpet hemocyanin hemocyanin
- PBS-T: Phosphat gepufferte Kochsalzlösung mit Zusatz von 0,1 % Tween 20
- ELISA: Enzyme-Linked-Immunosorbent-Assay
- mAb: Monoklonaler Antikörper (monoclonal antibody)
- MEIA: Micropartikel-Enzym-Immuno-Assay
- mM: mMo3ILiter
- PBS: Phosphat-gepufferte Kochsalzlösung
- tβhCG: throphoblastäre beta-Untereinheit des humanen Choriongonadotropin

### Zitierte Nicht-Patentliteratur:

(1) J.C.Pierce, T.F.Parsons, Annu.Rev.Biochem., 50 (1981) 465-495
(2) P.A.Rothman, V.A.Chao, M.R. Taylor et al," Mol.Reprod.Dev., 33 (1992) 1-6
(3) S.Dimhofer, M.Hermann, et al., J.Clin.Endocrinol.Metab., 81 (1996) 4212-4217
(4) Z.M.Lei, P.Toth, C.V.Rao und D.Pridham, J.Clin.Endocrino Metab.,77 (1993) 863-972
(5) T.Yokotani, T.Koizumi, R.Taniguchi et al., Int.J.Cancer, 71 (1997) 539-544
(6) P.Berger, W.Kranewitier, S.Madersbacher et al., FEBS Lett., 343 (1994) 229-233
(7) D.Beilet, V.Lazar, I.Bieche et al., Cancer Res., 57 (1997) 516-523
(8) I.Marcilliac, F.Troalen, J.-M.Bidart et al., Cancer Res., 52 (1992) 3901-3907
(9) HAlfthan, C.Haglund, J.Dabek et al., Clin.Chem., 38 (1992) 1981-1987
(10) V.Lazar, S.G.Diez, A.Laurent et al., Cancer Res., 55 (1995) 3735-3738
(11) P.N.Span, C.M.G.Thomas, J.J.Heuvel et al.,J.Endocrinol., 172 (2002) 489-495
(12) M.Lundin, S.Nordling, J.Lundin et al., Int.J.Cancer, 95 (2001) 18-22
(13) K.Hotakainen, B.Ljungberg, A.Paju et al., Brit.J.Cancer, 86 (2001) 185-189
(14) D.S.Hoon, T.Sarantou, F.Doiet al., Int.J.Cancer, 69 (1996) 369-374
(15) M.Bo undI.J.Boime, J.Biol.Chem., 267 (1992) 3179-3184
(16) K-Talmadge, N.C.Vamvakopoulus und J.C.Fiddes, Nature, 307 1984) 37-40
(17) P.Policastro, C.Ovitt, M.Hoshina et al., J.Biol.Chem., 258 (1983) 11492-11499
(18) P.Berger, C.Sturgeon, J.M.Bidart et al, Tumor Biol., 23 (2001) 1-38
(19) J.M.Bidart, S.Birken, P.Berger et al., Scand.J.Clin.Lab Invest., 216 (1993) 118-136
(20) P.Berger, J.M.Bidait, P.S.Delves et al., Mol.Cell Endocrinol,125 (1996) 33-43
(21) S.Dirnhofer, S.Madersbacher, J.M.Bidart et al., J.Endocrinol.,141 (1994) 153-162
(22) P.Berger, R.Klieber, W.Panmuong et al., J.Endocrinol., 125 (1990) 301-309
(23) J.M.Bidart, F.Troalen, C.J.Bohuon et al, J.Biol.Chem., 262 (1987) 15483-15489
(24) J.M.Bidart, D.H.Bellet, G.F.Alberici et al., Mol.Immunol., 24 (1987) 339-345
(25) P.K.Hotakainen, B.M.Serlachius et al., Mol.Cell.Endocrinol., 162 (2000) 79-85
(26) A.K.Miller-Lindholm, C.J.Labenz, J.Ramey et al., Endocrinology, 138 (1997) 5459-5465
(27) S.Madersbacher, C.Kratzik, R.Gerth et al., Cancer Res., 54 (1994) 5096-5100
(28) R Oyasu, L.Nan, P.Smith et al., Arch-Pathol.Lab.Med., 119 (1994) 715-717
(29) L.A.Cole, Clin.Chem., 43 (1997) 2233-2243
(30) L.A.Cole, J. Reprod. Med., 43 (1998) 3-10
(31) L.A.Cole, K.M.Rinne, S.Shahabi et al., Clin. Chem., 45 (1999) 313-314
(32) A.Kardana, M.M.Elliot, M.A.Gawinowicz et al., Endocrinology, 129 (1991) 1541-1550
(33) S.Birken A.Krichevsky, J.O'Connor et al., Endocrine, 10 (1999) 137-144
(34) A.Krichevsky, S.Birken, J.O'Connor et al., Endocrinology, 134 (1994) 1139-1145
(35) G.Kovalevskaya, S.Birken, T.Kakuma et al., J. Endocrinol., 161 (2000) 99-109
(36) P.Mock, G.Kovalevskaya, J.F.O'Connor et al., Hum. Reprod.,15 (2000) 2209-2214
(37) P.Mock, P.Bischof, R.Rivest et al., Hum. Reprod., 13 (1998) 2629-2632
(38) M.Seppälä, E.-M.Rutanen, et al., J.Clin.Endocrinol. Metab., 47 (1978) 1216-1219
(39) R W.Sharpe, W.Wrixon, et al., J.Clin.Endocrinol.Metab.,45 (1977) 496-499
(45) G-Kovalevskaya, S.Birken, T.Kakuma, et al., Clin.Chem., 45 (1999) 68-77
(46) C.Stähli, T.Staehelin, V.Miggiano et al., J.Immunol.Method., 32 (1980) 297-304
(47) G.Kovalevskaya, S.Birke, J.O'Connor et al., Endocrine, 3 (1995) 881-887
(48) J.B.Conde, A.B.Moreno, C.A.Bas, Hybridoma and Hybridomics 21 (2002) 3881-384
(49) RB.Marrifield, J.Am.Chem.Soc., 85 (1963) 2149-2154
(50) A.M.Krieg, Am.Rev.Immunol., 20 (2002) 709-760
(51) G.Köhler, C,Milestein, Nature, 256 (1975) 495
(52) S.Madersbach, P.Berger, Methods, 21 (2000) 41-50
(53) Pierce, Rockford, USA: EZ Antibody Production and Purification Kit, Carboxy Reactive
(54) L.A.Cole, Gynecol.Oncol., 71 (1998) 325-329

## Patentansprüche

1. Verfahren zur Bestimmung von definierten Zuständen oder Veränderungen in der Utsrusschleimhant oder im Epithel anderer Organe, bei dem in einer Körperflüssigkeitsprobe und/oder Gewebeprobe und/oder Zellen die Konzentration von humanen endomtrialem Choriongonadotropin (eßhCG/ehCG) und/oder nicht-trophoblastärem hCG (hCG Typ I, ß6, β7) spezifisch bestimmt wird.

2. Verfahren nach Anspruch 1, **dadurch gekennzeichnet, dass** auch die Konzentration von trophoblastärem hCG (hCG Typ II tβhCG) oder Gesamt-βhCG oder Gesamt-hCG bestimmt wird.

3. Verfahren nach Anspruch 1 oder 2, **dadurch gekennzeichnet, dass** die Bestimmung der Konzentration von endometrialem hCG (eßhCG/ehCG) mit mindestens einem Antikörper erFolgt, der spezifisch endometriales hCG (eßhCG/ehCG) and nicht trophoblastäres hCG (hCG Typ II, tßhCG erkennt.

4. Verfahren nach Anspruch 3, **dadurch gekennzeichnet, dass** der Antikörper spezifisch ein Peptid ausgewählt aus den Peptidsequenzen gemäß SEQ ID NO.1 oder 3 oder deren Teilsequenzen erkennt

5. Verfahren nach einem der Ansprüche 1 bis 4 **dadurch gekennzeichnet, dass** die Bestimmung der Konzentration von endometrialem hCG und gegebenenfalls trophoblastärem hCG oder Gesamt-ßhCG oder Gesamt-hCG in einer Probe in Form von Sekreten, Perfusionsflüssigkeit, Zellen oder Gewebe erfolgt, wobei diese aus peripherem Blut, Serum, Lochia, Menstrualblut, Fruchtwasser, Urin, Speichel, Augenkammerwasser, dem Urogenital- (insbesondere Uterus, Zervix, Vaginalproben), dem Gastrointestinal- (insbesondere Munschleimhaut, dem Respirationstrakt oder dem zentralen Nervensystem (insbesondere Liquor) stammen.

6. Verwendung eines Verfahrens nach einem der Ansprüche 1 bis 5 zur Bestimmung der Aufnahmebereitschaft der Uterusschleimhaut für eine befruchtete Eizelle in einer prospektiven oder retrospektiven Embryo-Implantationsdiagnostik.

7. Verwendung eines Verfahrens nach einem der Ansprüche 2 bis 5 zur Diagnose einer Schwangerschaft, eines frühen Schwangerschaftsverlustes, einer Extrauteringravidität des Schwangerschaftsverlaufs, von Schwangerschaftsstörungen, insbesondere zur *Differenzierung zwischen Störung des Endometriums/Dezidua von einer trophoblastären/einbryonalen Störung, zur Diagnose eines drohenden oder in Gang befindlichen Aborts, des Geburtsbeginns, für das Frühgeburten-Screening, zur Diagnose der Lochia oder Dezidua nach gestörter Schwangerschaft oder zur Beurteilung der Effektivität einer kontrazeptiven Methode.

8. Verwendung eines Verfahrens nach einem der Ansprüche 2 bis 5 zur Diagnose eines physiologischen Aufbaus eines Epithels oder einer beginnenden Dedifferanzierung oder einer beginnenden karzinomatösen Entartung oder eines Karzinoms und/oder zur Diagnose des "Phantom hCGs".

9. Antikörper, der spezifisch endometriales hCG (eßhCG/ehCG) und nicht trophoblastäres hCG (hCG Typ II tßhCG) erkennt, **dadurch gekennzeichnet, dass** er spezifisch eine Peptidsequenz gemäß SEQ ID Nr. 3 oder deren Teilsequenzen erkennt.

10. Testkit zur Bestimmung von definierten Zuständen oder Veränderungen in der Uterusschleimhaut oder im Epithel anderer Organe enthaltend mindestens einen Antikörper gemäß Anspruch 9 sowie weiterer Antikörper und Standards.

11. Peptid ausgewählt aus den Aminosäuresequenzen gemäß SEQ ID No. 14 und 3.

12. Verwendung eines Antikörpers gemäß Anspruch 9 oder eines Testkits nach Anspruch 10 oder von Peptiden gemäß Anspruch 11 zur Bestimmung von definierten Zuständen oder Veränderungen in der Uterusschleimhaut oder im Epithel anderer Organe, insbesondere zur Schwangerschaftsdiagnose oder zur Diagnose der Aufnahmebereitschaft der Uterusschleimhaut für eine befruchtete Eizelle in einer prospektiven oder retrospektiven Embryo-Implantatsdiagnosik, zur Diagnose einer Schwangerschaft, eines frühen Schwangerschaftsverlustes, einer Extrauteringravidität, des Schwangerschaftsverlaufs, von Schwangerschaftsstörungen insbesondere zur Differenzierung zwischen Störung des Endometriums/Dezidua von einer trophoblastären/embryonalen Störung zur Diagnose eines drohenden oder in Gang befindlichen Aborts, des Geburtysbeginns, für das Frühgeburten-Screening, zur Diagnose der Lochia oder Dezidua nach gestörter Schwangerschaft oder zur Beurteilung der Effektivität einer kontrazeptiven Methode, zur Diagnose eines physiologischen Anfbaus eines Epithels oder einer beginnenden Dedifferanzierung oder einer beginnenden karzinomatösen Entartung oder eines Karzinoms und/oder zur Diagnose des Phantom-hCGs.

13. Verwendung der endometrialen β-Untereinheit von humanem Chonongonadotropin (eβhCG) mit einer Aminosäuresequenz gemäß SEQ ID No. 10 oder der Gensequenz gemäß SEQ ID No. 7 als Marker für den gesunden Aufbau und Funktion des Endometriums oder der Dezidua, insbesondere in der Schwangerschaftsdiagnose oder der Diagnose der Aufnahmebereitschaft der Uterusschleimhaut für eine befruchtete Eizelle, in einer prospektiven oder retrospektiven Embryo-Implantatsdiagnostik, zur Diagnose einer Schwangerschaft, eines frühen Schwangerschaftsverlustes, einer Exrtrauteringravidität, des Schwangerschaftsverlaufs, von Schwangerschaftsstörungen, insbesondere zur Differenzierung zwischen Störung des Endometriums/Dezidua von einer trophoblastären/embryonalen Störung, zur Diagnose eines drohenden oder in Gang befindlichen Aborts, des Geburtsbeginns, für das Frühgeburten-Screening, zur Diagnose der Lochia oder Dezidua nach gestörter Schwangerschaft oder zur Beurteilung der Effektivität einer kontrazeptiven Methode, zur Diagnose eines physiologischen Aufbaus eines Epithels oder einer beginnenden Dedifferenzierung oder einer beginnenden karzinomatösen Entartung oder eines Karzinoms und/oder zur Diagnose des Phantom-hCGs.

## Claims

1. A method for determining defined states or modifications in the mucous membrane of the uterus or in the epithelium of other organs, wherein the concentration of human endometrial chorionic gonadotropin (eßhCG/ehCG) and/or non-trophoblastic hCG (hCG type I, ß6, ß7) are determined specifically in a body liquid sample and/or tissue sample and/or cells.

2. A method according claim 1, **characterized in that** the concentration of trophoblastic hCG (hCG type II, tßhCG) or total-ßhCG or total-hCG are additionally determined.

3. A method according to claim 1 or 2, **characterized in that** the determination of the concentration of endometrial hCG (eßhcG/ehcG) is performed with at least one antibody that specifically recognizes endometrial hCG (eßhCG/ehCG) and not trophoblastic hCG (hCG type II, tßhCG).

4. A method according to claim 3, **characterized in that** the antibody recognizes a peptide selected from the peptide sequences according to SEQ ID NO. 1 or SEQ ID NO. 3 or fragments thereof specifically.

5. A method according to anyone of claims 1 to 4, **characterized in that** the determination of the concentration of endometrial hCG and trophoblastic hCG or total-ßhCG or total-hCG is performed in a sample selected from the group of secretions, perfusion liquids, cells or tissues, wherein these are selected from peripheral blood, serum, lochia, menstrual blood, amniotic liquor, urine, salvia, eye chamber water, the urogenital system (especially uterus, cervix, samples from the vagina), the gastrointestinal system (especially oral mucosa), the respiratory tract or the central nervous system (especially liquor).

6. Use of a method according to any of claims 1 to 5 for determining receptivity of the mucous membrane of the uterus for a fertilized egg in the prospective or retrospective embryo implantation diagnostics.

7. Use of a method according to anyone of claims 2 to 5 for pregnancy, an early pregnancy loss, an extra-uterine pregnancy, success of pregnancy, pregnancy dysfunctions, especially for differentiation between dysfunction of the endometrium/dezidua versus trophoblastic/embryonic dysfunction, for diagnosing of an impending or forth going abort, onset of labor, for premature birth screening, for diagnosing the lochia or dezidua after dysfunction of pregnancy or for evaluating the effectiveness of a contraceptive method.

8. Use of a method according to any of claims 2 to 5 for diagnosing physiological states of the epithelium or a beginning dedifferentiation or a beginning carcinogenic degeneration or a carcinoma and/or for diagnosing of "phantom-hCGs".

9. An antibody that specifically recognizes endometrial hCG (eßhCG/ehCG) and not trophoblastic hCG (hCG type II, tßhcG), **characterized in that** the antibody specifically recognizes a peptide sequence according to SEQ ID NO. 3 or fragment thereof.

10. A test kit for determining defined states or modifications in the mucous of the uterus or in the epithelium of other organs comprising at least one antibody according to claim 9 as well as further antibodies and standards.

11. A peptide selected from the amino acid sequences according to SEQ ID NO. 14 and 3.

12. Use of an antibody according to claim 9 or a test kit according to claim 10 or a peptide according to claim 11 for determining defined states or modifications in the mucous of the uterus or in the epithelium of other organs, including for diagnose of pregnancy or for diagnosis of the receptiveness of the mucous of the uterus for a fertilized egg in prospective or retrospective embryo implantation diagnostics, for diagnosing a pregnancy, an early pregnancy loss, an extra uterine pregnancy, success of pregnancy, dysfunctions of pregnancy, including differenting between a dysfunction of the endometrium/dezidua versus a trophoblastic/embryonic dysfunction, for diagnosing of an impending or on going abort, the onset of labor, for the premature birth screening, for diagnosing the lochia or dezidua after dysfunction of pregnancy, or for evaluating the effectiveness of a contraceptive method, for diagnosing of the physiological states of an epithelium or a beginning dedifferentiation or beginning carcinogenic degeneration or carcinoma and/or for diagnosing of "phantom-hCGs".

13. Use of an endometrial β-subunit of human choriongonadotropin (eβCG) with an amino acid sequence according to SEQ ID No. 10 or the gene sequence according to SEQ ID NO. 7 as a marker for a healthy state and function of the endometrium or dezidua, including the use in diagnosing pregnancy or diagnosing the receptiveness of the mucous of the uterus for a fertilized egg in prospective or retrospective embryo implantation diagnostics, for diagnosis of pregnancy, an early pregnancy loss, an extra-uterine pregnancy, the success of pregnancy, the dysfunction of pregnancy, including for differentiation between a dysfunction of the endometrium/dezidua versus trophoblastic/embryonic dysfunction, for diagnosing an impending or on going abort, the onset of labor, for premature birth-screening, for diagnosing the lochia or dezidua after dysfunction of pregnancy or for evaluating the effectiveness of a contraceptive method, for diagnosing a physiological state of an epithelium or a beginning dedifferentiation or a beginning carcinogenic degeneration or carcinoma and/or for diagnosing of "phantom-hCGs".

## Revendications

1. Procédé de détermination d'états définis ou de modifications de la muqueuse utérine ou de l'épithélium d'autres organes, dans lequel dans un échantillon de fluide corporel et/ou un échantillon de tissu et/ou des cellules, la concentration de gonadotrophine chorionique humaine endométriale (eβhCG/ehCG) et/ou de hCG non trophoblastique (hCG de type I, β6, β7) est déterminé spécifiquement.

2. Procédé selon la revendication 1, **caractérisé en ce que** la concentration de hCG trophoblastique (hCG de type II, tβhCG) ou de βhCG totale ou de hCG totale est déterminée.

3. Procédé selon la revendication 1 ou 2, **caractérisé en ce que** la détermination de la concentration de hCG endométriale (eβhCG/ehCG) s'effectue avec au moins un anticorps qui reconnaît spécifiquement la hCG endométriale (eβhCG/ehCG) et non pas la hCG trophoblastique (hCG de type II, tβhCG).

4. Procédé selon la revendication 3, **caractérisé en ce que** l'anticorps reconnaît spécifiquement un peptide choisi parmi les séquences peptidiques selon SEQ ID NO. 1 ou 3 ou leurs séquences partielles.

5. Procédé selon l'une des revendications 1 à 4, **caractérisé en ce que** la détermination de la concentration de hCG endométriale et éventuellement, de hCG trophoblastique ou βhCG totale ou hCG totale s'effectue dans un échantillon sous la forme de sécrétions, de fluide de perfusion, de cellules ou de tissus, ceux-ci provenant de sang périphérique, de sérum, de lochies, de sang menstruel, de liquide amniotique, d'urine, de salive, d'humeur aqueuse de la chambre antérieure de l'oeil, d'échantillons urogénitaux (en particulier, de l'utérus, du col de l'utérus, du vagin), d'échantillons gastro-intestinaux (en particulier de muqueuse buccale, des voies respiratoires ou du système nerveux central (en particulier, LCR).

6. Utilisation d'un procédé selon l'une des revendications 1 à 5, pour la détermination de la capacité de réception par la muqueuse utérine d'un ovule fécondé, dans un diagnostic implantatoire prospectif ou rétrospectif de l'embryon.

7. Utilisation d'un procédé selon l'une des revendications 2 à 5, pour le diagnostic d'une grossesse, d'un avortement spontané précoce, d'une grossesse extra-utérine, de l'évolution de la grossesse, de troubles de la grossesse, en particulier pour la différenciation entre un trouble de l'endomètre/la caduque et un trouble trophoblastique/embryonnaire, pour le diagnostic d'une menace d'avortement ou d'avortement en cours, de début de l'accouchement, pour le dépistage des naissances prématurées, pour le diagnostic des lochies ou de la caduque après une grossesse perturbée ou pour la détermination de l'efficacité d'un procédé contraceptif.

8. Utilisation d'un procédé selon l'une des revendications 2 à 5 pour le diagnostic de la constitution physiologique d'un épithélium ou un début de dédifférenciation ou un début de dégénérescence carcinomateux ou d'un carcinome et/ou du diagnostic de "hCG fantôme".

9. Anticorps qui reconnaît spécifiquement la hCG endométriale (eβCG/ehCG) et non pas la hCG trophoblastique (hCH de type II, tβhCG), **caractérisé en ce qu'**il reconnaît spécifiquement une séquence peptidique selon SEQ ID NO. 3 ou ses séquences partielles.

10. Kit de test pour la determination d'états définis ou de modifications de la muqueuse utérine ou de l'épithélium d'autres organes, contenant au moins un anticorps selon la revendication 9 et d'autres anticorps et normes.

11. Peptide choisi parmi les séquences d'acides aminés selon SEQ ID NO. 14 et 3.

12. Utilisation d'un anticorps selon la revendication 9 ou d'un kit de test selon la revendication 10, ou de peptides selon la revendication 11 pour la détermination d'états définis ou de modifications dans la muqueuse utérine ou de l'épithélium d'autres organes, en particulier pour le diagnostic de la grossesse ou pour le diagnostic de la capacité de réception par la muqueuse utérine d'un ovule fécondé dans un diagnostic implantatoire prospectif ou rétrospectif de l'embryon, pour le diagnostic d'une grossesse, d'un avortement spontané précoce, d'une grossesse extra-utérine, de l'évolution de la grossesse, de troubles de la grossesse, en particulier pour la différenciation entre un trouble de l'endomètre/caduque et un trouble trophoblastique/embryonnaire, pour le diagnostic d'une menace d'avortement ou d'avortement en cours, de début de l'accouchement, pour le dépistage des naissances prématurées, pour le diagnostic des lochies ou de la caduque après une grossesse perturbée ou pour la détermination de l'efficacité d'un procédé contraceptif, pour le diagnostic de la constitution physiologique d'un épithélium ou d'un début de dédifférenciation ou un début de dégénérescence carcinomateux ou d'un carcinome et/ou du diagnostic de "hCG fantôme".

13. Utilisation de la sous-unité bêta endométriale de la gonadotrophine chorionique humaine (eβCG) ayant une séquence d'acides aminés selon SEQ ID NO. 10 ou une séquence de gène selon SEQ ID NO. 7 comme marqueur de la constitution saine et de la fonction de l'endomètre ou de la caduque, en particulier dans le diagnostic de la grossesse ou pour le diagnostic de la capacité de réception par la muqueuse de l'utérus d'un ovule fécondé dans un diagnostic implantatoire prospectif ou rétrospectif de l'embryon, pour le diagnostic d'une grossesse, d'un avortement spontané précoce, d'une grossesse extra-utérine, de l'évolution de la grossesse, de troubles de la grossesse, en particulier pour la différenciation entre un trouble de l'endomètre/caduque et un trouble trophoblastique/embryonnaire, pour le diagnostic d'une menace d'avortement ou d'avortement en cours, de début de l'accouchement, pour le dépistage des naissances prématurées, pour le diagnostic des lochies ou de la caduque après une grossesse perturbée ou pour la détermination de l'efficacité d'un procédé contraceptif, pour le diagnostic de la constitution physiologique d'un épithélium ou d'un début de dédifférenciation ou un début de dégénérescence carcinomateux ou d'un carcinome et/ou du diagnostic de "hCG fantôme".


### Sequenzprotokoll - Sequence Listing

<110> Universität Leipzig
<120> Verfahren und Mittel zur Bestimmung von bestimmten Zuständen bzw. Veränderungen im Uterusepithel und in Epithelien anderer Organe
<130> 401P07PCT
<150> DE10325639.3
   <151> 2003-06-06
   <150> DE1032563B.5
   <151> 2003-06-06
<160> 15
<210> 1
   <211> 15
   <212> PRT
   <213> artificial
   <220>
   <223> Epitope ß9 (eßhCG)
   <400> 1
   Thr Cys Asp Asp Pro Arg Phe Gln Ala Ser Ser Ser Ser Lys Ala
   1               5                   10                  15
<210> 2
   <211> 15
   <212> PRT
   <213> artificial
   <220>
   <223> Epitope ß9 (tβhCG) .
   <400> 2
   Thr Cys Asp Asp Pro Arg Phe Gln Asp Ser Ser Ser Ser Lys Ala
   1               5                   10                  15
<210> 3
   <211> 15
   <212> PRT
   <213> artificial
   <220>
   <223> Epitope eß1 (eßhCG)
   <400> 3
   Ser Arg Glu Met Leu Arg Pro Arg Cys Arg Pro Ile Asn Ala Thr
   1               5                   10                  15
<210> 4
   <211> 15
   <212> PRT
   <213> artificial
   <220>
   <223> Epitope β1 (tßhCG)
   <400> 4
   Ser Lys Glu Pro Leu Arg Pro Arg Cys Arg Pro Ile Asn Ala Thr
   1               5                   10                  15
<210> 5
   <211>. 861
   <212> DNA
   <213> human
   <220>
   <223> βhCG β7 cDNA-Sequenz
   <400> 5
<210> 6
   <211> 861
   <212> DNA
   <213> human
   <220>
   <223> βhCG β6 cDNA-Sequenz
   <400> 6
<210> 7
   <211> 861
   <212> DNA
   <213> human
   <220>
   <223> eßhCG ("endo" β6e) cDNA-Sequenz
   <400> 7
m: c,a r: g,a s: g, c y: t,o
<210> 8
   <211> 165
   <212> PORT
   <213> human
   <220>
   <223> tβhCG β5, β8, β3 (prehormone)
   <400> 8
   Met Glu Met Phe Gln Gly Leu Leu Leu Leu Leu Leu Leu Ser Met Gly
   -20                 -15                 -10                 -5
   Gly Thr Trp Ala Ser Lys Glu Pro Leu Arg Pro Arg Cys Arg Pro Ile
               -1   1              5                   10
   Asn Ala Thr Leu Ala Val Glu Lys Glu Gly Cys Pro Val Cys Ile Thr
           15                  20                  25
   Val Asn Thr Thr Ile Cys Ala Gly Tyr Cys Pro Thr Met Met Arg Val
       30                  35                  40
   Gly Val Leu Gln Leu Pro Ala Leu Pro Gln Val Val Cys Asn Tyr Arg
   45                  50                  55                  60
   Asp Val Arg Phe Glu Ser Ile Arg Leu Pro Gly Cys Pro Arg Gly Val
                   65                  70                  75
   Asn Pro Val Val Ser Tyr Ala Val Ala Leu Ser Cys Gln Cys Ala Leu
               80                  85                  90
   Cys Arg Arg Ser Thr Thr Asp Cys Gly Gly Pro Lys Asp His Pro Leu
           95                  100                 105
   Thr Cys Asp Asp Pro Arg Phe Gln Asp Ser Ser Ser Ser Lys Ala Pro
       110                 115                 120
   Pro Pro Ser Leu Pro Ser Pro Ser Arg Leu Pro Gly Pro Ser Asp Thr
   125                 130                 135                 140
   Pro Ile Leu Pro Gln
                   145
<210> 9
   <211> 165
   <212> PRT⁻
   <213> human
   <220>
   <223> βhCG β7 (preharmone)
   <400> 9
   Met Glu Met Phe Gln Gly Leu Leu Leu Leu Leu Leu Leu Ser Met Gly
   -20 -15 -10 -5
   Gly Thr Trp Ala Ser Arg Glu Met Leu Arg Pro Arg Cys Arg Pro Ile
   -1 1 5 10
   Asn Ala Thr Leu Ala Val Glu Lys Glu Gly Cys Pro Val Cys Ile Thr
   15 20 25
   Val Asn Thr Thr Ile Cys Ala Gly Tyr Cys Pro Thr Met Met Arg Val
   30 35 40
   Gly Val Leu Gln Leu Pro Ala Leu Pro Gln Val Val Cys Asn Tyr Arg
   45 50 55 60
   Asp Val Arg Phe Glu Ser Ile Arg Leu Pro Gly Cys Pro Arg Gly Val
   65 70 75
   Asn Pro Val Val Ser Tyr Ala Val Ala Leu Ser Cys Gln Cys Ala Leu
   80 85 90
   Cys Arg Arg Ser Thr Thr Asp Cys Gly Gly Pro Lys Asp His Pro Leu
   95 100 105
   Thr Cys Asp Asp Pro Arg Phe Gln Ala Ser Ser Ser Ser Lys Ala Pro
   110 115 120
   Pro Pro Ser Leu Pro Ser Pro Ser Arg Leu Pro Gly Pro Ser Asp Thr
   125 130 135 140
   Pro Ile Leu Pro Gln
   145
<210> 10
   <211> 165
   <212> PRT
   <213> human
   <220>
   <223> eßhCG β6e (with Arg in Pos 2) (prehormone)
   <400> 10
   Met Glu Met Phe Gln Gly Leu Leu Leu Leu Leu Leu Leu Ser Met Gly
   -20                 -15                 -10                 -5
   Gly Thr Trp Ala Ser Arg Glu Met Leu Arg Pro Arg Cys Arg Pro Ile
               -1  1               5                   10
   Asn Ala Thr Leu Ala Val Glu Lys Glu Gly Cys Pro Val Cys Ile Thr
           15                  20                  25
   Val Asn Thr Thr Ile Cys Ala Gly Tyr Cys Pro Thr Met Met Arg Val
       30                  35                  40
   Gly Val Leu Gln Leu Pro Ala Leu Pro Gln Val Val Cys Asn Tyr Arg
   45                  50                  55                  60
   Asp Val Arg Phe Glu Ser Ile Arg Leu Pro Gly Cys Pro Arg Gly Val
                   65                  70                  75
   Asn Pro Val Val Ser Tyr Ala Val Ala Leu Ser Cys Gln Cys Ala Leu
               80                  85                  90
   Cys Arg Arg Ser Thr Thr Asp Cys Gly Gly Pro Lys Asp His Pro Leu
           95                  100                 105
   Thr Cys Asp Asp Pro Arg Phe Gln Ala Ser Ser Ser Ser Lys Ala Pro
       110                 115                 120
   Pro Pro Ser Leu Pro Ser Pro Ser Arg Leu Pro Gly Pro Ser Asp Thr
   125                 130                 135                 140
   Pro Ile Leu Pro Gln
                   145
<210> 11
   <211> 141
   <212> PRT⁻
   <213> human
   >220>
   <223> βLH β4 (prehormone)
   <400> 11
   Met Glu Met Leu Gln Gly Leu Leu Leu Leu Leu Leu Leu Ser Met Gly
   -20 -15 -10 -5
   Gly Ala Trp Ala Ser Arg Glu Pro Leu Arg Pro Trp Cys His Pro Ile
   -1 +1 5 10
   Asn Ala Ile Leu Ala Val Glu Lys Glu Gly Cys Pro Val Cys Ile Thr
   15 20 25
   Val Asn Thr Thr Ile Cys Ala Gly Tyr Cys Pro Thr Met Met Arg Val
   30 35 40
   Leu Gln Ala Val Leu Pro Pro Leu Pro Gln Val Val Cys Thr Tyr Arg
   45 50 55 60
   Asp Val Arg Phe Glu Ser Ile Arg Leu Pro Gly Cys Pro Arg Gly Val
   65 70 75
   Asp Pro Val Val Ser Phe Pro Val Ala Leu Ser Cys Arg Cys Ala Pro
   80 85 90
   Cys Arg Arg Ser Thr Ser Asp Cys Gly Gly Pro Lys Asp His Pro Leu
   95 100 105
   Thr Cys Asp His Pro Glu Leu Ser Gly Leu Leu Phe Leu
   110 115
<210> 12
   <211> 10
   <212> PRT
   <213> artificial
   <220>
   <223> Peptide P1 (eßhCG)
   z00> 12
<210> 13
   <211> 10
   <212> PRT
   <213> artificial
   <220>
   <223> Peptide K1 (tβhCG)
   <400> 13
   Cys Asp Asp Pro Arg Phe Gln Asp Ser Ser
   1 5 10
<210> 14
   <211> 11
   <212> PRT
   <213> artificial
   <220>
   <223> Peptide P2 (eßhCG)
   <400> 14
   Ser Arg Glu Met Leu Arg Pro Arg Cys Arg Pro
   1 5 10
<210> 15
   <211> 11
   <212> PRT
   <213> artificial
   <220>
   <223> Peptide K2 (tβhCG)
   <400> 15
   Ser Lys Glu Pro Leu Arg Pro Arg Cys Arg Pro
   1 5 10 11